# EUROPEAN PATENT APPLICATION

(11) **EP 3 799 851 A1**
(43) Date of publication of application: **07.04.2021**
(21) Application number: 19870105.4
(22) Date of filing: 08.10.2019
(51) Int. Cl.: A61H 3/00, A61F 5/01

(54) **WALKING ASSISTANCE MECHANISM AND WALKING ASSISTANCE DEVICE**

(30) Priority: 09.10.2018 JP 2018191220
(71) Applicant: Uchida Co., Ltd., Saitama, 354-0045 (JP)
(72) Inventor: HARA Katsuyuki, Iruma-gun, Saitama 354-0045 (JP)
(74) Representative: TBK
(86) International application number: PCT/JP2019/039697
(87) International publication number: WO 2020/075723

(57) **Abstract**

A walking assistance mechanism of the present invention includes: a waist side link; a thigh side link connected to the waist side link to be able to relatively swing in a front-rear direction of the user by means of a hip joint side swinging shaft; a lower leg side link connected to the thigh side link to be able to relatively swing in the front-rear direction by means of a knee joint side swinging shaft; a knee extension posture maintenance mechanism that regulates relative swinging between the thigh side link and the lower leg side link to maintain a knee extension posture in which a relative angle between the thigh side link and the lower leg side link is large; and a knee bending posture allowance mechanism that releases the regulation of the knee extension posture maintenance mechanism to allow for a knee bending posture in which the relative angle between the thigh side link and the lower leg side link becomes smaller when the thigh side link is positioned on a more rearward side than a position at which the thigh side link forms a predetermined reference relative angle with respect to the waist side link. According to the present invention, the walking assistance mechanism that allows a knee on a leg of a pedestrian on the front side to maintain an extended state and allow a knee on a leg of the pedestrian on the rear side to be bent while walking can be provided.

## Description

### Technical Field

The present invention relates to a walking assistance mechanism and a walking assistance device for assisting the walking of a user.

### Background Art

A walking assistance system including a belt assembly, an exotendon connected to the belt assembly, and a leg frame capable of joint motion, for example, has been proposed in the conventional techniques as a walking assistance system for assisting walking (see WO2012/125765, for example). Such a leg frame is provided with a knee pulley, so that a user can bend the knee. Such an exotendon is configured to be capable of expanding and contracting by means of a cable and a spring. The cable of the exotendon is wound also around the leg frame including the knee pulley. Elastic energy of the spring is transmitted to the leg frame through the cable. When the leg of the user is positioned on the rear side, the spring is expanded to accumulate elastic energy. When the leg of the user moves forward, the spring contracts to release the elastic energy. Such elastic energy facilitates the forward movement of the leg of the user.

### Summary of Invention

### Technical Problem

Since a paraplegic person cannot put his or her strength into the lower half of the body, such a person may fall down if both legs are bent while walking. The above-described walking assistance system, however, allows for the knee bending state of both legs. Thus, it is difficult for a paraplegic person to use the above-described walking assistance system.

In view of the foregoing circumstances, it is an object of the present invention to provide a walking assistance mechanism and a walking assistance device that allow a knee on a leg of a pedestrian on the front side to maintain an extended state and allow a knee on a leg of the pedestrian on the rear side to be bent while walking.

### Solution to Problem

The present invention has been made to solve the above-described problem. A walking assistance mechanism of the present invention is a walking assistance mechanism for assisting walking of a user in a front-rear direction, including: a waist side link disposed near a waist portion of the user; a thigh side link disposed along a thigh portion of the user and connected to the waist side link to be able to relatively swing in the front-rear direction of the user by means of a hip joint side swinging shaft; a lower leg side link disposed along a lower leg portion of the user and connected to the thigh side link to be able to relatively swing in the front-rear direction by means of a knee joint side swinging shaft; a knee extension posture maintenance mechanism configured to regulate relative swinging between the thigh side link and the lower leg side link to maintain a knee extension posture in which a relative angle between the thigh side link and the lower leg side link is large; and a knee bending posture allowance mechanism configured to release the regulation of the knee extension posture maintenance mechanism to allow for a knee bending posture in which the relative angle between the thigh side link and the lower leg side link becomes smaller when the thigh side link is positioned on a more rearward side than a position at which the thigh side link forms a predetermined reference relative angle with respect to the waist side link.

In the walking assistance mechanism of the present invention, the knee extension posture maintenance mechanism includes: a lower leg side regulation shaft that is provided at a position away from the knee joint side swinging shaft in the lower leg side link and that is configured to move integrally with the lower leg side link; a thigh side regulation shaft that is provided at a position away from the knee joint side swinging shaft in the thigh side link and that is configured to move integrally with the thigh side link; a first regulation side link provided to be able to relatively swing with respect to the lower leg side link using the lower leg side regulation shaft as a swinging axis; an intermediate regulation shaft provided at a position away from the lower leg side regulation shaft in the first regulation side link; a second regulation side link that is provided to be able to relatively swing with respect to the thigh side link using the thigh side regulation shaft as a swinging axis and that is connected to the first regulation side link via the intermediate regulation shaft to be able to relatively swing; and a stopper mechanism configured to delimit an upper limit of a relative opening angle between the first regulation side link and the second regulation side link to a braking posture in which the relative opening angle, representing a degree of opening between the first regulation side link and the second regulation side link using the intermediate regulation shaft as a reference, is 180 degrees, or larger than or equal to 180 degrees. A four bar linkage is constituted by the knee joint side swinging shaft, the lower leg side regulation shaft, the thigh side regulation shaft, and the intermediate regulation shaft. The thigh side link and the lower leg side link assume a knee extension posture when the first regulation side link and the second regulation side link assume the braking posture. The thigh side link and the lower leg side link assume a knee bending posture when the relative opening angle between the first regulation side link and the second regulation side link becomes smaller than 180 degrees.

In the walking assistance mechanism of the present invention, the knee extension posture maintenance mechanism includes a biasing part configured to bias the first regulation side link and/or the second regulation side link in a direction in which the relative opening angle between the first regulation side link and the second regulation side link increases.

In the walking assistance mechanism of the present invention, the knee extension posture maintenance mechanism includes a biasing part configured to bias the lower leg side link in a direction in which the thigh side link and the lower leg side link assume a knee extension posture.

In the walking assistance mechanism of the present invention, the knee bending posture allowance mechanism includes: a connected body provided in at least any one of the first regulation side link, the second regulation side link, and the intermediate regulation shaft; and a moving mechanism configured to change the relative opening angle between the first regulation side link and the second regulation side link to be smaller than 180 degrees by moving the connected body.

In the walking assistance mechanism of the present invention, the moving mechanism moves the connected body along with a change in a relative angle of the thigh side link with respect to the waist side link.

In the walking assistance mechanism of the present invention, the moving mechanism includes an interlocking member with one thereof being connected to the waist side link and the other thereof being connected to the connected body. When the thigh side link is positioned on a more rearward side than the position at which the thigh side link forms the predetermined reference relative angle with respect to the waist side link, the interlocking member moves the connected body to make the relative opening angle between the first regulation side link and the second regulation side link smaller than 180 degrees.

In the walking assistance mechanism of the present invention, the interlocking member is a wire, and the connected body is moved as a result of the wire pulling the connected body.

In the walking assistance mechanism of the present invention, a contact part configured to change a path of the interlocking member by coming into contact with the interlocking member is disposed in a manner radially displaced from the hip joint side swinging shaft. The interlocking member is relatively moved with respect to the thigh side link as a result of the contact part moving closer to or farther away from the waist side link along with a relative swinging operation between the waist side link and the thigh side link, and the path of the interlocking member is thereby changed.

In the walking assistance mechanism of the present invention, the moving mechanism includes a winding mechanism configured to wind the interlocking member along with a relative swinging operation between the waist side link and the thigh side link. The interlocking member is in a state wound by the winding mechanism when the thigh side link is positioned on a more rearward side than the position at which the thigh side link forms the predetermined reference relative angle with respect to the waist side link.

In the walking assistance mechanism of the present invention, the winding mechanism includes: a lever member configured to rotate about the hip joint side swinging shaft, to which one end of the interlocking member is fixed; an engagement member configured to swing about the hip joint side swinging shaft together with the thigh side link and engage with the lever member; and a rotation regulating part configured to regulate rotation of the lever member. The lever member is rotated by being pressed by the swinging of the engagement member. When the rotation of the lever member is regulated by the rotation regulating part, the engagement member has a phase difference with the lever member, and the interlocking member is thereby wound by the lever member.

In the walking assistance mechanism of the present invention, the lower leg side link includes a guide part extending in a guide direction that approaches the thigh side link as moving toward an upper side from a lower side in a height direction of the user when the thigh side link and the lower leg side link assume a knee extension posture. The lower leg side regulation shaft engages with the guide part in a manner movable along the guide direction.

In the walking assistance mechanism of the present invention, a wearing part to be worn in the waist portion of the user is included. The wearing part includes: a waist side belt part to be worn around the waist portion of the user; and a crotch portion side belt part to be worn so as to support the user from a lower side in a crotch portion of the user.

A walking assistance device of the present invention is a walking supporting device including any one of the above-described walking assistance mechanisms one for each leg of the user. The walking supporting device includes: a connecting member configured to integrally connect the waist side links in the two walking assistance mechanisms; a bridging link bridged between the two walking assistance mechanisms and connected to the two thigh side links; and a bridging link guide part configured to guide vicinities of ends of the bridging link in the front-rear direction. A moving direction of one end of the bridging link becomes opposite to a moving direction of the other end of the bridging link along with operations of the respective walking assistance mechanisms in the front-rear direction.

### Advantageous Effects of Invention

According to the walking assistance mechanism and the walking assistance device of the present invention, there is obtained an advantageous effect such that while walking, a knee on a leg of a pedestrian on the front side is allowed to maintain an extended state and a knee on a leg of the pedestrian on the rear side is allowed to bend.

### Brief Description of Drawings

Fig. 1 is a front view of a walking assistance device according to a first embodiment of the present invention.
Fig. 2 is a front view illustrating a user wearing the walking assistance device according to the first embodiment of the present invention.
Fig. 3 is a side view of the walking assistance device according to the first embodiment of the present invention.
Figs. 4(A) to 4(C) are side views illustrating walking assistance mechanisms arranged in chronological order to show states when a leg moves from a front side toward a rear side in the walking assistance device according to the first embodiment of the present invention.
Fig. 5(A) is a diagram showing a braking posture state in a knee extension posture maintenance mechanism of the walking assistance device according to the first embodiment of the present invention. Fig. 5(B) is a diagram showing a state in which the braking posture is released to assume a protruded posture in the knee extension posture maintenance mechanism of the walking assistance device according to the first embodiment of the present invention.
Fig. 6 is a diagram illustrating a modified example of a braking posture in the knee extension posture maintenance mechanism of the walking assistance device according the first embodiment of the present invention.
Fig. 7(A) is a diagram showing a braking posture state in the knee extension posture maintenance mechanism of the walking assistance device according to the first embodiment of the present invention. Fig. 7(B) is a diagram showing a state in which the braking posture is released to assume a protruded posture in the knee extension posture maintenance mechanism of the walking assistance device according to the first embodiment of the present invention.
Fig. 8(A) is a diagram illustrating a modified example of a moving range limiting part in the knee extension posture maintenance mechanism of the walking assistance device according to the first embodiment of the present invention. Fig. 8(B) is a diagram illustrating another modified example of the moving range limiting part in the knee extension posture maintenance mechanism of the walking assistance device according to the first embodiment of the present invention.
Fig. 9(A) is a side view illustrating an upper side, in a height direction, of the walking assistance device according to the first embodiment of the present invention than a thigh side link. Fig. 9(B) is a side view illustrating a lower side, in the height direction, of the walking assistance device according to the first embodiment of the present invention than the thigh side link.
Figs. 10(A) and 10(B) are side views, each illustrating the upper side, in the height direction, of the walking assistance mechanism than the thigh side link, arranged in chronological order to show states when the leg moves from the front side toward the rear side in the walking assistance device according to the first embodiment of the present invention.
Figs. 11(A) and 11(B) are side views, each illustrating the upper side, in the height direction, of the walking assistance mechanism than the thigh side link, arranged in chronological order to show states when the leg moves from the front side toward the rear side in a modified example of the walking assistance device according to the first embodiment of the present invention.
Fig. 12 is a diagram illustrating another modified example of the walking assistance device according to the first embodiment of the present invention.
Figs. 13(A) to 13(C) are side views, each illustrating the upper side, in the height direction, of the walking assistance mechanism than the thigh side link, arranged in chronological order to show states when the leg moves from the front side toward the rear side in another modified example of the walking assistance device according to the first embodiment of the present invention.
Fig. 14(A) is a back view illustrating the upper side, in the height direction, of the walking assistance device according to the first embodiment of the present invention than the thigh side link. Fig. 14(B) is a schematic plan view of the walking assistance device according to the first embodiment of the present invention.
Fig. 15 is a side view showing how the user wearing the walking assistance device according to the first embodiment of the present invention is walking.

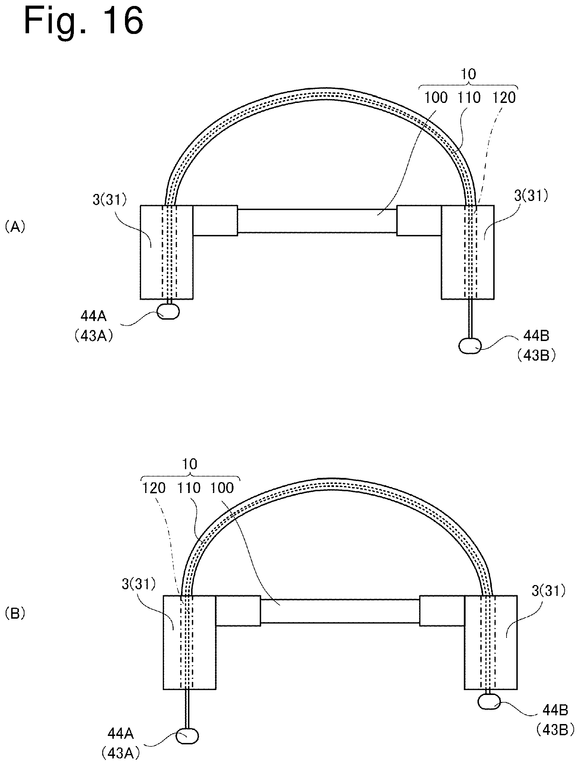

Fig. 16(A) is a schematic plan view showing a state in which one of the walking assistance mechanisms is advanced toward the front side and the other one of the walking assistance mechanisms is positioned on the rear side in the walking assistance device according to the first embodiment of the present invention. Fig. 16(B) is a schematic plan view showing a state in which the one of the walking assistance mechanisms is positioned on the rear side and the other one of the walking assistance mechanisms is advanced toward the front side in the walking assistance device according to the first embodiment of the present invention.
Fig. 17 is a diagram showing a relationship between time T and an angle γ when the user of the walking assistance device according to the first embodiment of the present invention makes walking action.
Fig. 18 is a front view of a walking assistance device according to a second embodiment of the present invention.
Fig. 19 is a front view illustrating a user wearing the walking assistance device according to the second embodiment of the present invention.
Fig. 20(A) is a plan view illustrating a wearing part of the walking assistance device according to the second embodiment of the present invention. Fig. 20(B) is a plan view illustrating the wearing part when a user with large thighs wears the walking assistance device according to the second embodiment of the present invention.
Fig. 21(A) is a perspective view illustrating a state before a second belt part is connected to an assist part by a connecting mechanism in the walking assistance device according to the second embodiment of the present invention. Fig. 21(B) is a plan view illustrating a state when the second belt part is connected to the assist part by the connecting mechanism in the walking assistance device according to the second embodiment of the present invention.
Fig. 22 is a side view illustrating the walking assistance device according to the second embodiment of the present invention.
Fig. 23 is a side view illustrating a knee extension posture maintenance mechanism in the walking assistance device according to the second embodiment of the present invention.
Figs. 24(A) and 24(B) are side views arranged in chronological order to show how a four bar linkage in the walking assistance device according to the second embodiment of the present invention is operated while maintaining a knee extension posture.
Figs. 25(A) and 25(B) are side views arranged in chronological order to show how the four bar linkage in the walking assistance device according to the second embodiment of the present invention is operated to assume a knee bending posture.
Fig. 26 is a side view showing a state in which the four bar linkage in the walking assistance device according to the second embodiment of the present invention is operated to achieve the maximum knee bending.
Figs. 27(A) to 27(C) are side views illustrating walking assistance mechanisms arranged in chronological order to show states when a leg moves from the front side toward the rear side in the walking assistance device according to the second embodiment of the present invention.
Fig. 28(A) is a side view of the walking assistance device when a relative angle θ made by a thigh side link with respect to a waist side link in the walking assistance device according to the second embodiment of the present invention is θ2. Fig. 28(B) is a side view of the walking assistance device when the relative angle θ made by the thigh side link with respect to the waist side link in the walking assistance device according to the second embodiment of the present invention is a reference relative angle θ1.

### Description of Embodiments

Embodiments of the present invention will be described below with reference to the accompanying drawings.

### First Embodiment:

A walking assistance device 1 according to a first embodiment of the present invention is provided for assisting the walking of a user. The walking assistance device 1 includes, for example, a wearing part 2, a lateral pair of waist side links 3, a lateral pair of thigh side links 4, a lateral pair of hip joint side swinging shafts 5, a lateral pair of lower leg side links 6, a lateral pair of knee joint side swinging shafts 7, a lateral pair of knee extension posture maintenance mechanisms 8, a lateral pair of knee bending posture allowance mechanisms 9, and a walking posture ensuring part 10. Among these, the waist side link 3, the thigh side link 4, the hip joint side swinging shaft 5, the lower leg side link 6, the knee joint side swinging shaft 7, the knee extension posture maintenance mechanism 8, and the knee bending posture allowance mechanism 9 together constitute a walking assistance mechanism 1A. In the walking assistance device 1, a lateral pair of such walking assistance mechanisms 1A are disposed on the right and left sides of the user.

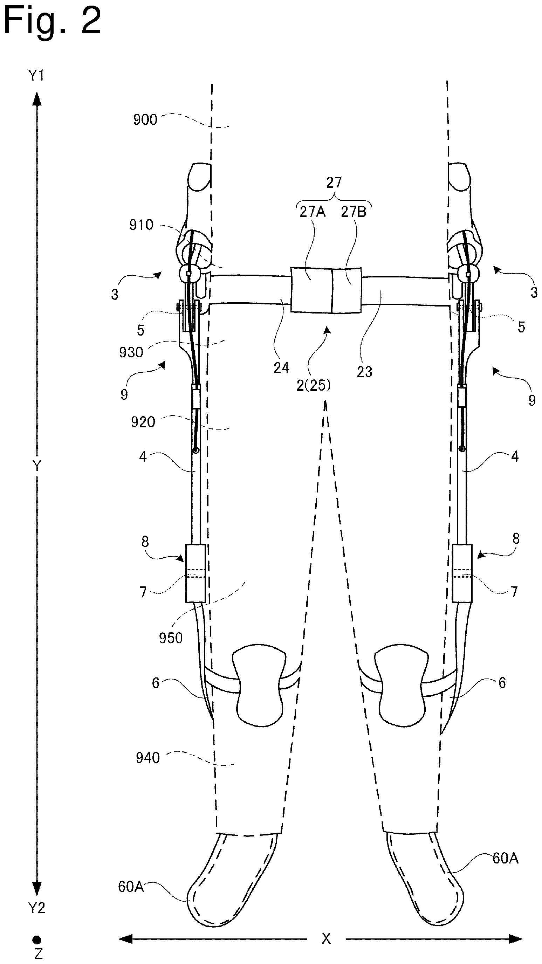

As shown in Fig. 2, the front-rear direction of a user 900 wearing the walking assistance device 1 is assumed to be the front-rear direction of the walking assistance device 1, and hereinafter referred to simply as a front-rear direction Z. The height direction of the user 900 wearing the walking assistance device 1 is assumed to be the height direction of the walking assistance device 1, and hereinafter referred to simply as a height direction Y. The width direction of the user 900 wearing the walking assistance device 1 is assumed to be the width direction of the walking assistance device 1, and hereinafter referred to simply as a width direction X.

### Wearing Part:

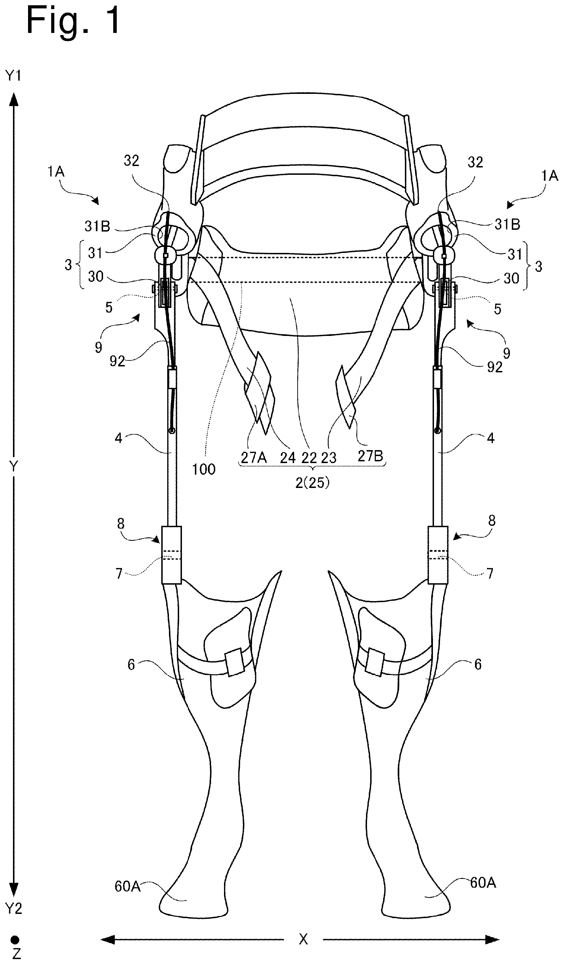

The wearing part 2 will now be described with reference to Figs. 1 and 2. As shown in Fig. 2, the wearing part 2 is worn around a waist portion 910 of the user 900 by being wrapped around the waist portion 910 of the user 900. As shown in Fig. 1, the wearing part 2 is constituted by a waist side belt part 25. The waist side belt part 25 is worn by being wrapped around the waist portion 910 of the user 900. The waist side belt part 25 includes, for example, a lower back side belt portion 22, a first belly side belt portion 23, a second belly side belt portion 24, a connecting mechanism 27, and a length adjustment part (not shown).

The lower back side belt portion 22 covers the waist portion 910 of the user 900. The lower back side belt portion 22 is attached to a waist side connecting member 100 to be described later, or those disposed around the lower back side belt portion 22 such as the waist side links 3. Specifically, the length direction of the lower back side belt portion 22 extends in the width direction X of the walking assistance device 1 between the lateral pair of waist side links 3, and end portions of the lower back side belt portion 22 in the length direction thereof are folded back toward the front (Z2) side in the front-rear direction Z of the walking assistance device 1. The lower back side belt portion 22 is formed in a strip shape, for example. The lower back side belt portion 22 is chiefly made of a material having flexibility. Specific aspects of the lower back side belt portion 22 include, for example, a lower back side belt portion woven in a sheet shape with a synthetic resin such as nylon, and a lower back side belt portion obtained by enwrapping a sponge formed in a sheet shape with a cover.

As shown in Fig. 1, the first belly side belt portion 23 and the second belly side belt portion 24 are each formed in a strip shape. The strip width of the first belly side belt portion 23 and the second belly side belt portion 24 is smaller than the strip width of the lower back side belt portion 22. The first belly side belt portion 23 and the second belly side belt portion 24 are chiefly made of a material having flexibility. By way of example, specific aspects of the first belly side belt portion 23 and the second belly side belt portion 24 include those woven in a sheet shape with a synthetic resin such as nylon, those obtained by enwrapping a sponge formed in a sheet shape with a cover, and those made of a low resilience material.

As shown in Fig. 1, the first belly side belt portion 23 and the second belly side belt portion 24 are connected to intermediate portions, or both ends, of the lower back side belt portion 22 so as to extend in the length direction of the lower back side belt portion 22 starting from the both ends (the lateral ends in Fig. 1) of the lower back side belt portion 22 in the length direction thereof. That is, the first belly side belt portion 23 and the second belly side belt portion 24 extend from the both ends of the lower back side belt portion 22 in the length direction thereof toward the front (Z2) side (the belly side of the user 900) in the front-rear direction Z of the walking assistance device 1.

The connecting mechanism 27 is provided for connecting the first belly side belt portion 23 and the second belly side belt portion 24 together. The connecting mechanism 27 includes a first connection part 27A and a second connection part 27B. The first connection part 27A , the second connection part 27B are provided at leading ends of the first belly side belt portion 23 , the second belly side belt portion 24, respectively, or in the vicinity of such leading ends. The first connection part 27A and the second connection part 27B together have a structure capable of detachably connecting to each other. Examples of the connecting mechanism 27 include a connecting mechanism using Magic Tape (registered trademark), a connecting mechanism using a button, and an inserting part and a buckle to be locked with, and connected to, the inserting part when the inserting part is inserted thereinto. The first belly side belt portion 23 and the second belly side belt portion 24 are connected to each other by the connection between the first connection part 27A and the second connection part 27B.

When the user 900 wears the first belly side belt portion 23 and the second belly side belt portion 24, the first belly side belt portion 23 and the second belly side belt portion 24 are wrapped around the belly side as shown in Fig. 2. The first connection part 27A and the second connection part 27B are then connected together. At this time, the first connection part 27A and the second connection part 27B are located at the front side of the belly (lower belly portion) of the user 900. The wearing part 2 is thereby worn around the waist portion 910 of the user 900. When the connection between the first connection part 27A and the second connection part 27B is released, on the other hand, the first belly side belt portion 23 and the second belly side belt portion 24 wrapped around the waist portion 910 of the user 900 come off the belly of the user 900.

The length adjustment part (not shown) is provided for adjusting the length of the first belly side belt portion 23 and/or the second belly side belt portion 24 for constricting the belly side of the user 900. For example, the length adjustment part relatively moves the first connection part 27A and/or the second connection part 27B along the first belly side belt portion 23 and/or the second belly side belt portion. Configuring the first connection part 27A or the second connection part 27B in this manner substantially enables the length of the first belly side belt portion 23 and the second belly side belt portion 24 worn by the user 900 to be adjusted. Note that the length adjustment part is not limited to the configuration described above, but may have any other configuration.

### Waist Side Link:

The waist side link 3 will now be described with reference to Figs. 1 to 3. As shown in Fig. 2, the waist side link 3 is a link disposed near the waist portion 910 of the user 900. One waist side link 3 is disposed on each of the right and left sides of the user 900.

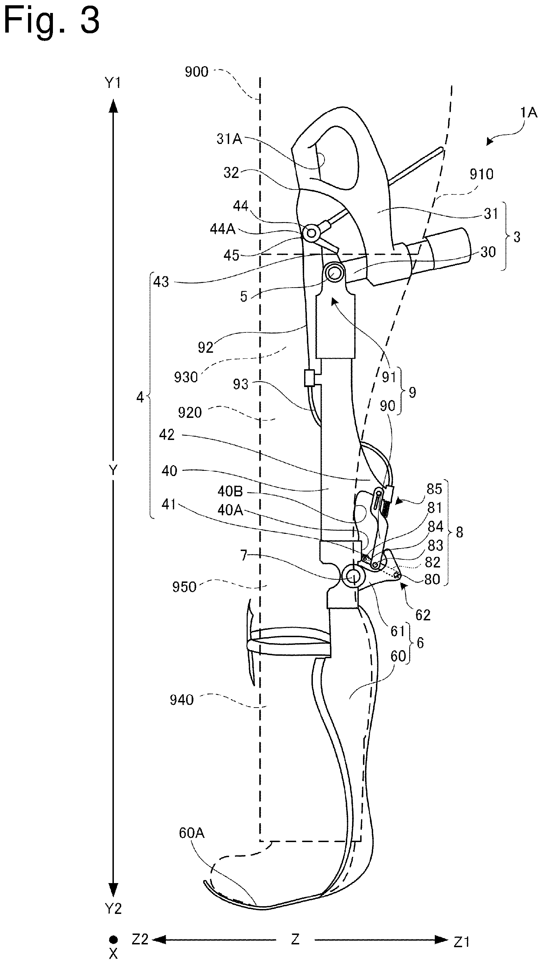

As shown in Figs. 1 and 3, the waist side link 3 includes a base link 30 and an assist part 31. The base link 30 is a link connected to the thigh side link 4. The base link 30 assumes a posture extending in the front-rear direction Z of the user 900.

As shown in Figs. 1 and 3, the assist part 31 is connected to the base link 30 while assuming a posture extending toward the upper (Y1) side in the height direction Y of the base link 30 assuming a posture extending in the front-rear direction Z. Note that the base link 30 and the assist part 31 may be integrally formed. The assist part 31 includes a grip portion 31A. The grip portion 31A is a portion to be gripped by a third person other than the user. The grip portion 31A is provided near the leading end of the assist part 31 on the upper (Y1) side in the height direction Y.

### Thigh Side Link and Hip Joint Side Swinging Shaft:

The thigh side link 4 and the hip joint side swinging shaft 5 will now be described with reference to Figs. 1 to 5. As shown in Figs. 2 and 3, the thigh side link 4 is disposed along a thigh portion 920 of the user 900. As shown in Fig. 3, the thigh side link 4 includes a thigh side link main body part 40, a first thigh side protruded part 41, a second thigh side protruded part 42, and a thigh side extended part 43.

The thigh side link main body part 40 is a portion of the thigh side link 4 corresponding to the lower (Y2) side in the height direction Y from the hip joint side swinging shaft 5. The thigh side link main body part 40 is constituted by a plate-shaped body having an elongated plate shape, for example. The plate-shaped body is disposed with the longitudinal direction thereof being substantially parallel to the direction in which the thigh portion 920 of the user 900 extends. The thigh side extended part 43 is a portion of the thigh side link 4 extended toward the upper (Y1) side in the height direction Y from the hip joint side swinging shaft 5.

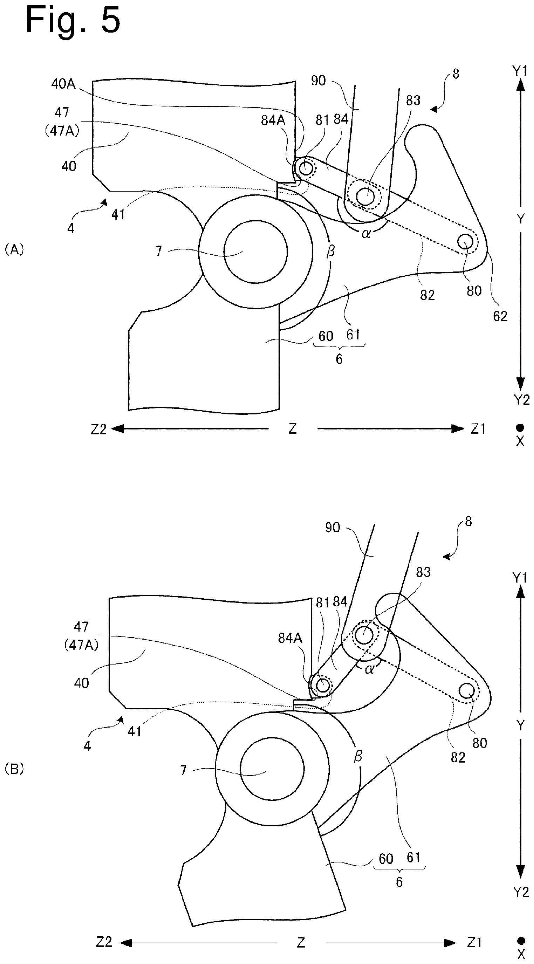

As shown in Figs. 5(A) and 5(B), the first thigh side protruded part 41 is a portion protruded toward the rear (Z1) side in the front-rear direction Z starting from a back surface 40A of the thigh side link main body part 40 near the knee joint side swinging shaft 7. As shown in Fig. 3, the second thigh side protruded part 42 is a portion protruded toward the rear (Z1) side in the front-rear direction Z starting from a back surface 40B of the thigh side link main body part 40 on the upper (Y1) side in the height direction Y than the first thigh side protruded part 41. The first thigh side protruded part 41 and the second thigh side protruded part 42 move integrally with the thigh side link main body part 40. The first thigh side protruded part 41 and the second thigh side protruded part 42 may be formed integrally with the thigh side link main body part 40, or may be connected to the thigh side link main body part 40 as separate members.

As shown in Fig. 3, the hip joint side swinging shaft 5 is disposed near a hip joint 930 of the user 900. The hip joint side swinging shaft 5 extends in the width direction X (the direction normal to the paper plane of Fig. 3).

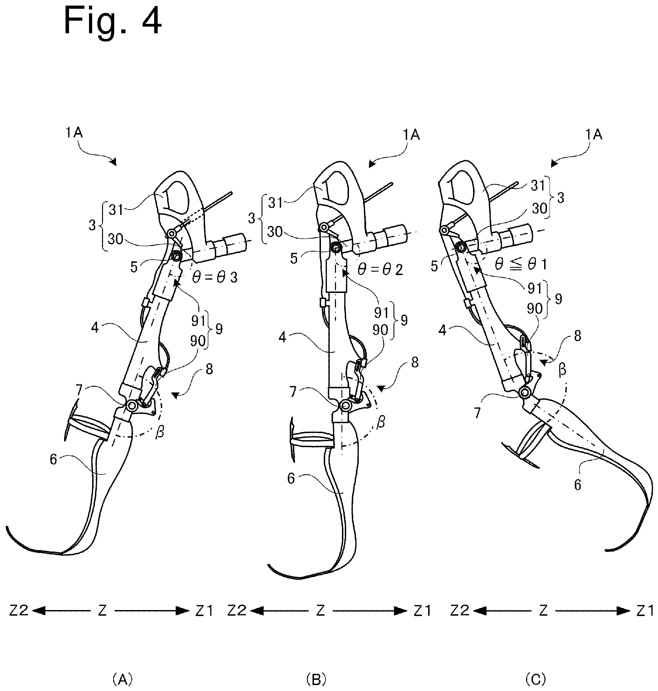

As shown in Fig. 3, the thigh side link 4 is connected to the waist side link 3 via the hip joint side swinging shaft 5. That is, the thigh side link 4 and the waist side link 3 are swingably connected to the hip joint side swinging shaft 5 using the hip joint side swinging shaft 5 as an axis. Thus, the thigh side link 4 can relatively swing in the front-rear direction Z with respect to the waist side link 3 as shown in Figs. 4(A) to 4(C).

### Lower Leg Side Link and Knee Joint Side Swinging Shaft:

The lower leg side link 6 and the knee joint side swinging shaft 7 will now be described with reference to Figs. 1 to 5. As shown in Figs. 2 and 3, the lower leg side link 6 is disposed along a lower leg portion 940 of the user 900. As shown in Fig. 3, the lower leg side link 6 is constituted by a lower leg side link main body part 60 and a lower leg side protruded part 61.

The lower leg side link main body part 60 is formed, for example, by a planar-shaped planar member covering from below the knee through the calf to the sole on the back surface side of the lower leg portion 940 of the user 900. When the user 900 wears the walking assistance device 1, the sole of the lower leg portion 940 is placed on a placement surface 60A of the above-described planar member (the lower leg side link main body part 60). The lower leg side protruded part 61 is a portion protruded toward the rear (Z1) side in the front-rear direction Z starting from the lower leg side link main body part 60 substantially near the knee joint side swinging shaft 7, for example. Note that the lower leg side protruded part 61 may be protruded from any other portion in the lower leg side link main body part 60.

The lower leg side protruded part 61 moves integrally with the lower leg side link main body part 60. Note that the lower leg side protruded part 61 may be formed integrally with the lower leg side link main body part 60, or may be connected to the lower leg side link main body part 60 as a separate member.

As shown in Fig. 3, the knee joint side swinging shaft 7 is disposed near a knee joint 950 of the user 900. The knee joint side swinging shaft 7 extends in the width direction X (the direction normal to the paper plane of Fig. 3).

As shown in Fig. 3, the lower leg side link 6 is connected to the thigh side link 4 via the knee joint side swinging shaft 7. That is, the lower leg side link 6 is swingably connected to the knee joint side swinging shaft 7 using the knee joint side swinging shaft 7 as an axis. Thus, the lower leg side link 6 can relatively swing in the front-rear direction Z with respect to the thigh side link 4 as shown in Figs. 4(B) and 4(C).

### Knee Extension Posture Maintenance Mechanism:

The knee extension posture maintenance mechanism 8 will now be described with reference to Figs. 3 to 8. The knee extension posture maintenance mechanism 8 regulates relative swinging between the thigh side link 4 and the lower leg side link 6 to maintain a knee extension posture in which a relative angle β (see Figs. 4(A) to 4(C)) between the thigh side link 4 and the lower leg side link 6 is large. The knee extension posture refers to a posture of the thigh side link 4 and the lower leg side link 6 by which the knee of the user 900 wearing the walking assistance device 1 is extended. In the knee extension posture, the relative angle β between the thigh side link 4 and the lower leg side link 6 becomes approximately 180 degrees. As shown in Fig. 3, the knee extension posture maintenance mechanism 8 includes, for example, a lower leg side regulation shaft 80, a thigh side regulation shaft 81, a first regulation side link 82, an intermediate regulation shaft 83, a second regulation side link 84, and a stopper mechanism 85.

### Lower Leg Side Regulation Shaft:

As shown in Figs. 5(A) and 5(B), the lower leg side regulation shaft 80 is provided in the lower leg side link 6 on a more rearward (Z1) side in the front-rear direction Z than the knee joint side swinging shaft 7. Specifically, the lower leg side regulation shaft 80 is provided near a leading end 62 of the lower leg side protruded part 61 on the rear (Z1) side in the front-rear direction Z, for example. The lower leg side regulation shaft 80 moves integrally with the lower leg side protruded part 61. The lower leg side regulation shaft 80 extends in the width direction X (the direction normal to the paper plane of Figs. 5(A) and 5(B)).

### Thigh Side Regulation Shaft:

As shown in Figs. 5(A) and 5(B), the thigh side regulation shaft 81 is provided in the thigh side link 4 on a more rearward (Z1) side in the front-rear direction Z than the knee joint side swinging shaft 7. Specifically, the thigh side regulation shaft 81 is provided in the first thigh side protruded part 41 of the thigh side link 4. The thigh side regulation shaft 81 moves integrally with the thigh side link 4 (the first thigh side protruded part 41). The thigh side regulation shaft 81 extends in the width direction X.

The lower leg side protruded part 61 extends more toward the rear (Z1) side in the front-rear direction Z than the first thigh side protruded part 41. Thus, the thigh side regulation shaft 81 is located at a position closer to the knee joint side swinging shaft 7 than the lower leg side regulation shaft 80.

### First Regulation Side Link, Intermediate Regulation Shaft, and Second Regulation Side Link:

As shown in Figs. 5(A) and 5(B), the first regulation side link 82 is connected, substantially at one end thereof, to the lower leg side protruded part 61 (the lower leg side link 6) via the lower leg side regulation shaft 80 to be able to relatively swing with respect to the lower leg side protruded part 61 (the lower leg side link 6) using the lower leg side regulation shaft 80 as a swinging axis. The first regulation side link 82 is constituted by an elliptic cylindrical body, for example, but is not limited thereto. The first regulation side link 82 may be constituted by a body having any other shape.

The intermediate regulation shaft 83 is provided on a more forward (Z2) side in the front-rear direction Z than the lower leg side regulation shaft 80. The intermediate regulation shaft 83 is provided at a position closer to the knee joint side swinging shaft 7 than the lower leg side regulation shaft 80 and farther away from the knee joint side swinging shaft 7 than the thigh side regulation shaft 81 in the front-rear direction Z. That is, the intermediate regulation shaft 83 is provided in between the lower leg side regulation shaft 80 and the thigh side regulation shaft 81 in the front-rear direction Z. In the present embodiment, the intermediate regulation shaft 83 is provided near the other end of the first regulation side link 82, for example. The intermediate regulation shaft 83 extends in the width direction X (the direction normal to the paper plane of Figs. 5(A) and 5(B)).

The second regulation side link 84 is connected, substantially at one end thereof, to the first thigh side protruded part 41 (the thigh side link 4) via the thigh side regulation shaft 81 to be able to relatively swing with respect to the thigh side link 4 using the thigh side regulation shaft 81 as a swinging axis. The second regulation side link 84 is constituted by an elliptic cylindrical body, for example, but is not limited thereto. The second regulation side link 84 may be constituted by a body having any other shape. The second regulation side link 84 is also connected, substantially at the other end thereof, to the first regulation side link 82 via the intermediate regulation shaft 83 to be able to relatively swing. The intermediate regulation shaft 83 may be provided integrally with not the first regulation side link 82 but the second regulation side link 84, or the intermediate regulation shaft 83 may be inserted into shaft holes provided near the other ends of the first regulation side link 82 and the second regulation side link 84 as a member separated from the first regulation side link 82 and the second regulation side link 84.

### Four Bar Linkage:

In the present embodiment, a four bar linkage is formed by connecting the lower leg side protruded part 61 (the lower leg side link 6), the first regulation side link 82, the second regulation side link 84, and the first thigh side protruded part 41 (the thigh side link 4) to one another by means of the knee joint side swinging shaft 7, the lower leg side regulation shaft 80, the thigh side regulation shaft 81, and the intermediate regulation shaft 83 to be able to relatively swing as shown in Figs. 5(A) and 5(B).

When a connected body 90 connected to the first regulation side link 82 and the second regulation side link 84 by means of the intermediate regulation shaft 83 in a state shown in Fig. 5(A) is moved toward the upper (Y1) side in the height direction Y, for example, the intermediate regulation shaft 83 is also moved toward the upper (Y1) side in the height direction Y as shown in Fig. 5(B). At this time, the first regulation side link 82 swings toward the lower (Y2) side in the height direction Y (clockwise in Fig. 5(B)), and the second regulation side link 84 swings toward the lower (Y2) side in the height direction Y (counterclockwise in Fig. 5(B)). Consequently, the first regulation side link 82 and the second regulation side link 84 form a protrusion toward the upper (Y1) side in the height direction Y. Furthermore, the lower leg side protruded part 61 (the lower leg side link 6) at this time swings toward the upper (Y1) side in the height direction Y (counterclockwise in Fig. 5(B)) by being pulled by the first regulation side link 82.

When the connected body 90 in the state shown in Fig. 5(B) is moved toward the lower (Y2) side in the height direction Y, on the other hand, the intermediate regulation shaft 83 is also moved toward the lower (Y2) side in the height direction Y. At this time, the first regulation side link 82 swings toward the lower (Y2) side in the height direction Y (counterclockwise in Fig. 5(B)), and the second regulation side link 84 swings toward the lower (Y2) side in the height direction Y (clockwise in Fig. 5(B)). Thus, the first regulation side link 82 and the second regulation side link 84 have the state shown in Fig. 5(A), i.e., assuming a braking posture to be explained in the section of Stopper Mechanism to be described later. Furthermore, the lower leg side protruded part 61 (the lower leg side link 6) at this time swings toward the upper (Y1) side in the height direction Y (clockwise in Fig. 5(B)) by being pushed toward the lower (Y2) side in the height direction Y by the first regulation side link 82.

### Stopper Mechanism:

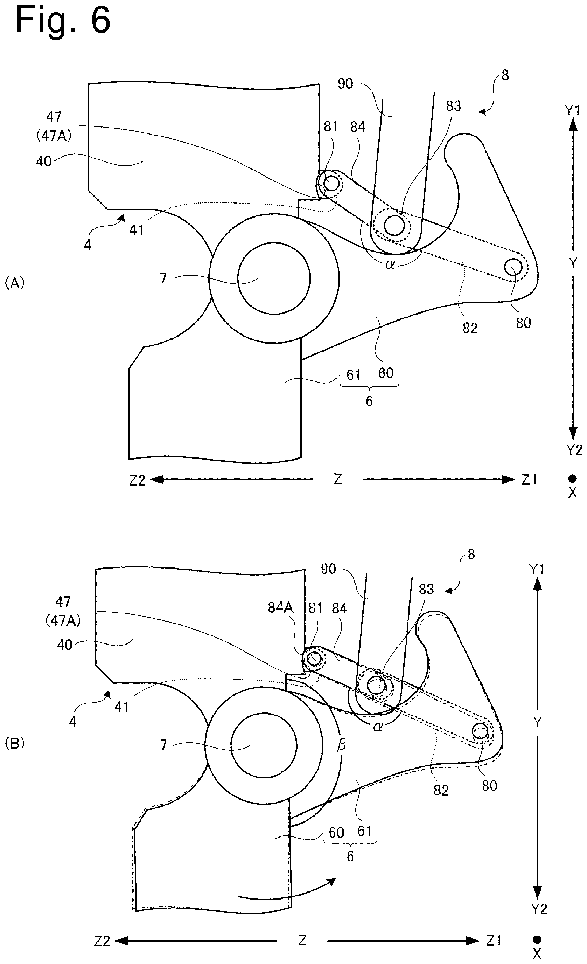

The stopper mechanism 85 will now be described with reference to Figs. 5 to 8. The stopper mechanism 85 is provided for causing the first regulation side link 82 and the second regulation side link 84 to have the braking posture. The braking posture refers to a posture of the first regulation side link 82 and the second regulation side link 84 that regulates the swinging of the lower leg side link 6 even when an external force to cause the lower leg side link 6 to swing toward the rear (Z1) side in the front-rear direction Z is applied to the lower leg side link 6. Specifically, the braking posture refers to, as shown in Figs. 5(A) and 6(A), a posture of the first regulation side link 82 and the second regulation side link 84 that allows a relative opening angle α, which represents a degree of opening between the first regulation side link 82 and the second regulation side link 84 using the intermediate regulation shaft 83 as a reference, to be 180 degrees (see Fig. 5(A)), or larger than or equal to 180 degrees (see Fig. 6(A)). The stopper mechanism 85 delimits the upper limit of the relative opening angle α between the first regulation side link 82 and the second regulation side link 84 to be 180 degrees, or larger than or equal to 180 degrees.

When the first regulation side link 82 and the second regulation side link 84 assume the braking posture as shown in Fig. 5(A), the first regulation side link 82 and the second regulation side link 84 serve as a prop between the thigh side link 4 and the lower leg side link 6, thereby regulating the swinging of the lower leg side link 6 with respect to the thigh side link 4. Consequently, the relative angle β between the thigh side link 4 and the lower leg side link 6 is kept approximately at 180 degrees, and the thigh side link 4 and the lower leg side link 6 thus assume the knee extension posture. The thigh side link main body part 40 includes a depressed portion 47 in the vicinity of the first thigh side protruded part 41. The depressed portion 47 is depressed toward the front (Z2) side in the front-rear direction Z starting from the back surface 40A, which also serves as the starting point of the first thigh side protruded part 41. In the state where the first regulation side link 82 and the second regulation side link 84 are assuming the braking posture, even when a force to cause the lower leg side link 6 to relatively swing toward the rear (Z1) side in the front-rear direction Z (counterclockwise in Fig. 6(B)) with respect to the thigh side link 4 is applied as shown in Fig. 6(B), a leading end portion 84A of the second regulation side link 84 interferes with a depressed surface 47A that constitutes the depressed portion 47 when the second regulation side link 84 swings about the thigh side regulation shaft 81. Thus, it is possible to regulate the relative swinging of the lower leg side link 6 with respect to the thigh side link 4. The depressed surface 47A that constitutes the depressed portion 47 may be a curved surface or a flat surface. Note that the lower leg side link 6, the first regulation side link 82 and the second regulation side link 84, etc. before the movement and those after the movement are drawn in an overlapped manner in Fig. 6(B). Those before the movement are indicated chiefly by dashed-dotted lines, whereas those after the movement are drawn chiefly by solid lines.

When the first regulation side link 82 and the second regulation side link 84 release the braking posture to have a protruded posture in which the first regulation side link 82 and the second regulation side link 84 form a protrusion toward the upper (Y1) side in the height direction Y as shown in Fig. 5(B), on the other hand, the first regulation side link 82 and the second regulation side link 84 no longer serve as a prop, thereby allowing the lower leg side link 6 to swing with respect to the thigh side link 4. Consequently, the relative angle β between the thigh side link 4 and the lower leg side link 6 becomes smaller than approximately 180 degrees as shown in Fig. 5(B), and the thigh side link 4 and the lower leg side link 6 thus assume a knee bending posture. Note that the knee bending posture refers to a posture of the thigh side link 4 and the lower leg side link 6 by which the knee of the user 900 wearing the walking assistance device 1 is bent. In the knee bending posture, the relative angle β between the thigh side link 4 and the lower leg side link 6 becomes smaller than approximately 180 degrees.

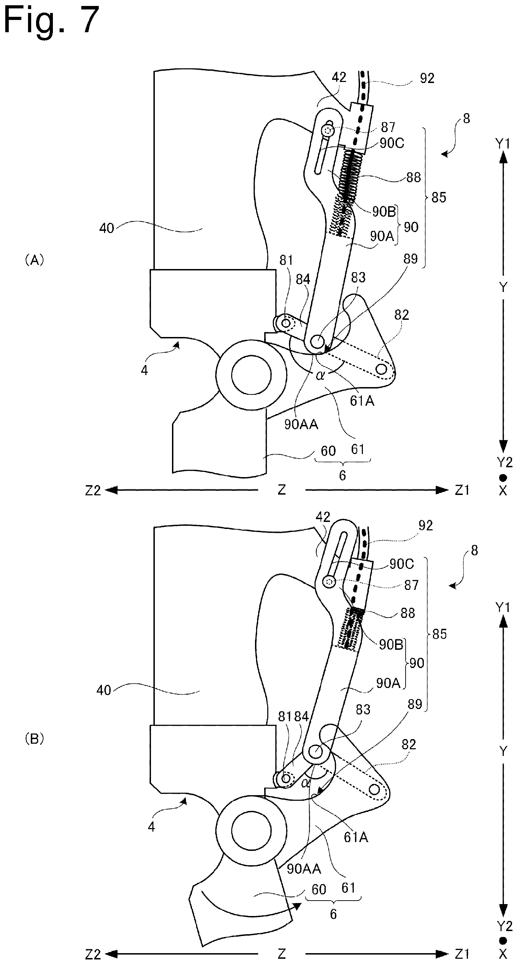

As shown in Figs. 7(A) and 7(B), the stopper mechanism 85 as described above includes, for example, the connected body 90, a guide part 87, a biasing part 88, and a moving range limiting part 89. The connected body 90 includes a connection part 90A and a guide side engagement part 90B.

As shown in Figs. 7(A) and 7(B), the connection part 90A constitutes part of the connected body 90, and is connected to the first regulation side link 82 and the second regulation side link 84 via the intermediate regulation shaft 83. The connection part 90A can relatively swing with respect to the first regulation side link 82 and the second regulation side link 84 using the intermediate regulation shaft 83 as an axis. Moreover, the connection part 90A extends in such a tilt direction that the connection part 90A becomes farther away from the thigh side link 4 (approaches the rear (Z1) side more in the front-rear direction Z) as approaching the upper (Y1) side in the height direction Y from the intermediate regulation shaft 83.

As shown in Figs. 7(A) and 7(B), the guide side engagement part 90B constitutes part of the connected body 90, and includes a through hole 90C. The through hole 90C is an elongated hole passing completely through the guide side engagement part 90B in the width direction X and extending for a predetermined distance in the extending direction of the connection part 90A (the above-described tilt direction). Note that the connection part 90A and the guide side engagement part 90B may be integrally formed, or may be formed as separate members.

The guide part 87 engages with the through hole 90C for guiding the connected body 90 in a guide direction. One example of such a guide direction in the present embodiment is the extending direction in which the through hole 90C extends (the above-described tilt direction), for example, but the guide direction is not limited thereto. Any other direction may be employed as long as such a direction allows the first regulation side link 82 and the second regulation side link 84 to assume the braking posture and the protruded posture.

As shown in Figs. 7(A) and 7(B), the guide part 87 is constituted by a shaft extending in the width direction X. Thus, the connected body 90 is guided in the guide direction swingably using the shaft functioning as the guide part 87 as a swinging axis. That is, the connected body 90 is guided in the guide direction of the through hole 90C while changing its posture. The shaft functioning as the guide part 87 is provided in the second thigh side protruded part 42 of the thigh side link 4.

As shown in Figs. 7(A) and 7(B), the biasing part 88 is provided for biasing the connected body 90 (the connection part 90A) in the guide direction. That is, even when the connected body 90 (the connection part 90A) moves toward the upper side (the side farther away from the lower leg side protruded part 61) in the guide direction, the biasing part 88 presses the connected body 90 (the connection part 90A) so that the connected body 90 moves toward the lower side (the side closer to the lower leg side protruded part 61) in the guide direction. Thus, without the application of an external force, the intermediate regulation shaft 83 is moved toward the side closer to the lower leg side protruded part 61 in the guide direction by the biasing part 88, thereby causing the first regulation side link 82 and the second regulation side link 84 to swing in a direction making the relative opening angle α larger. That is, the biasing part 88 operates to press the first regulation side link 82 and the second regulation side link 84 in the direction making the relative opening angle α larger. The biasing part 88 is constituted by a coil spring, for example. The coil spring is connected to both of the connected body 90 (the connection part 90A) and the second thigh side protruded part 42 while assuming a posture to expand and contract in the guide direction between the connected body 90 (the connection part 90A) and the second thigh side protruded part 42 (a state to press toward the side closer to the lower leg side protruded part 61 in the guide direction).

As shown in Figs. 7(A) and 7(B), the moving range limiting part 89 is provided for delimiting the moving range of the connection part 90A so that the first regulation side link 82 and the second regulation side link 84 assume the braking posture. The moving range limiting part 89 is constituted, for example, by a contact surface 61A of the lower leg side protruded part 61 capable of making contact with a leading end surface 90AA of the connection part 90A proximal to the lower leg side protruded part 61 when the connection part 90A moves toward the side closer to the lower leg side protruded part 61 in the guide direction. The leading end surface 90AA of the connection part 90A and the contact surface 61A of the lower leg side protruded part 61 face each other in the guide direction. When the contact surface 61A of the lower leg side protruded part 61 and the leading end surface 90AA of the connection part 90A are in contact with each other, the first regulation side link 82 and the second regulation side link 84 assume the braking posture.

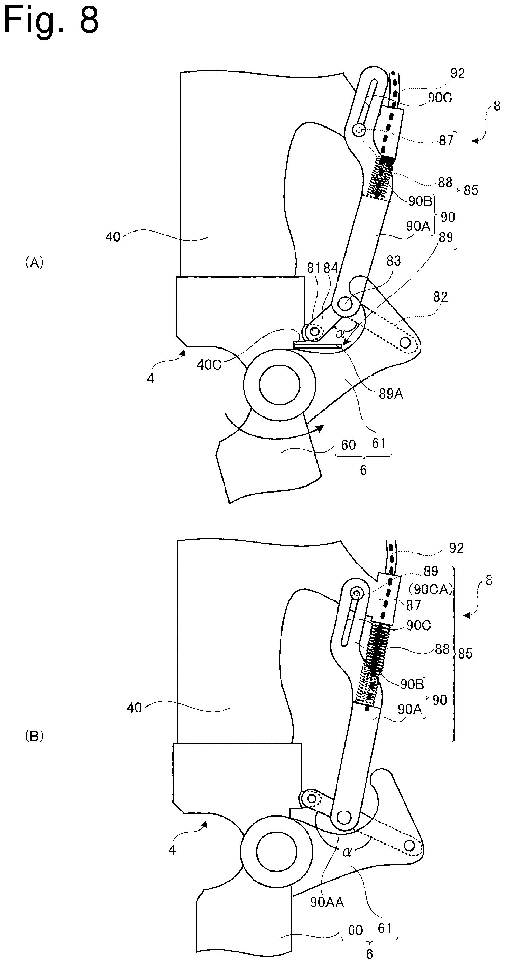

Alternatively, the moving range limiting part 89 may be constituted, for example, by a contact part 89A facing the leading end surface 90AA of the connection part 90A in the guide direction and capable of making contact with the leading end surface 90AA of the connection part 90A as shown in Fig. 8(A), which is provided separately from the contact surface 61A of the lower leg side protruded part 61. When the contact part 89A and the leading end surface 90AA of the connection part 90A come in contact with each other, the first regulation side link 82 and the second regulation side link 84 assume the braking posture. Note that the contact part 89A is constituted by a protruded part protruded toward the rear (Z1) side in the front-rear direction Z from a back surface 40C of the thigh side link main body part 40 between the depressed portion 47 of the thigh side link main body part 40 and the lower leg side protruded part 61 (in the height direction Y). Note that not the leading end surface 90AA of the connection part 90A but the first regulation side link 82 or the second regulation side link 84 may come into contact with the contact part 89A. When the first regulation side link 82 or the second regulation side link 84 is in contact with the contact part 89A, the first regulation side link 82 and the second regulation side link 84 assume the braking posture.

Alternatively, the moving range limiting part 89 may be constituted, for example, by an end face 90CA of the through hole 90C on the side distal to the leading end surface 90AA of the connection part 90A as shown in Fig. 8(B). In this case, when the connection part 90A moves toward the side closer to the lower leg side protruded part 61 in the guide direction, the end face 90CA of the through hole 90C and the shaft functioning as the guide part 87 come into contact with each other. This regulates a further movement of the connection part 90A toward the side closer to the lower leg side protruded part 61 in the guide direction. When the end face 90CA of the through hole 90C and the shaft functioning as the guide part 87 are in contact with each other, the first regulation side link 82 and the second regulation side link 84 assume the braking posture.

### Knee Bending Posture Allowance Mechanism:

The knee bending posture allowance mechanism 9 will now be described with reference to Figs. 1 to 11. The knee bending posture allowance mechanism 9 is provided for releasing the regulation of the knee extension posture maintenance mechanism 8 to allow for a knee bending posture in which the relative angle β between the thigh side link 4 and the lower leg side link 6 becomes smaller when the thigh side link 4 swings more toward the rear (Z1) side in the front-rear direction Z (the counterclockwise side in Fig. 4) than a position at which the thigh side link 4 forms a predetermined reference relative angle θ1 with respect to the waist side link 3 as shown in Fig. 4(C). An example of the predetermined reference relative angle θ1 is an angle satisfying θ1 < θ2, when a relative angle θ of the thigh side link 4 with respect to the waist side link 3 when the user is in a standing state and thus the thigh side link 4 is assuming a posture extending in the vertical direction is equal to a relative angle θ2 (see Fig. 4(B)), for example. Note that the predetermined reference relative angle θ1 shown in Fig. 4(C) is given merely as an example, and the degree of such a tilt is not limited to the state shown in Fig. 4(C).

For example, when the thigh side link 4 swings more toward the front (Z2) side in the front-rear direction Z (the clockwise side in Fig. 4) than the position at which the thigh side link 4 forms the predetermined reference relative angle θ1 with respect to the waist side link 3 as shown in Figs. 4(A) and 4(B), the relative angle β between the thigh side link 4 and the lower leg side link 6 is kept approximately at 180 degrees. Thus, the thigh side link 4 and the lower leg side link 6 assume the knee extension posture. That is, when the relative angle θ of the thigh side link 4 with respect to the waist side link 3 exceeds θ1, the thigh side link 4 and the lower leg side link 6 assume the knee extension posture.

When the thigh side link 4 swings more toward the rear (Z1) side in the front-rear direction Z (the counterclockwise side in Fig. 4) than the position at which the thigh side link 4 forms the predetermined reference relative angle θ1 with respect to the waist side link 3 as shown in Fig. 4(C), on the other hand, the regulation of the knee extension posture maintenance mechanism 8 is released. Consequently, the relative angle β between the thigh side link 4 and the lower leg side link 6 becomes smaller, and the thigh side link 4 and the lower leg side link 6 thus assume the knee bending posture. That is, when the relative angle θ of the thigh side link 4 with respect to the waist side link 3 is smaller than or equal to θ1, the thigh side link 4 and the lower leg side link 6 assume the knee bending posture.

The knee bending posture allowance mechanism 9 includes the connected body 90 (the connection part 90A) and a moving mechanism 91. Since the connected body 90 has already been described in the section of Stopper Mechanism, the description thereof will be omitted here.

The moving mechanism 91 is provided for moving the connected body 90 so that the relative opening angle α between the first regulation side link 82 and the second regulation side link 84 is changed to be smaller than 180 degrees. The connected body 90 is moved by the moving mechanism 91 along with a change in the relative angle θ of the thigh side link 4 with respect to the waist side link 3. When the relative angle θ of the thigh side link 4 with respect to the waist side link 3 is in the range of θ1 < θ (see Figs. 4(A) and 4(B)), the moving mechanism 91 keeps the connected body 90 unmoved as shown in Figs. 5(A) and 6(A). Consequently, the first regulation side link 82 and the second regulation side link 84 keep a state of having the braking posture, or return to positions to assume the braking posture. When the relative angle θ of the thigh side link 4 with respect to the waist side link 3 is in the range of θ1 ≥ θ (see Fig. 4(C)), the moving mechanism 91 moves the connected body 90 so that the first regulation side link 82 and the second regulation side link 84 assume the protruded posture (the posture to make the relative opening angle α smaller than 180 degrees) as shown in Fig. 5(B).

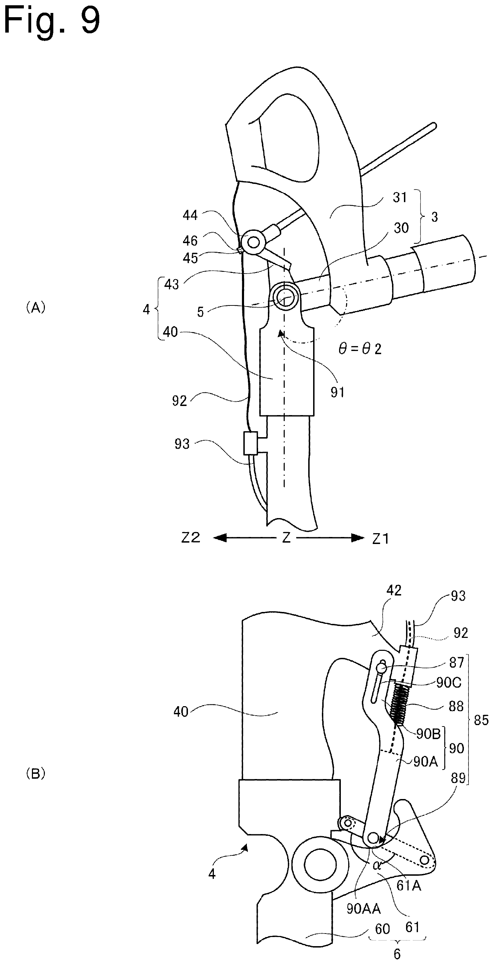

As shown in Figs. 9(A) and 9(B), the moving mechanism 91 includes an interlocking member 92, for example. One end of the interlocking member 92 is connected to the waist side link 3 (the assist part 31), and the other end thereof is connected to the connected body 90 (the connection part 90A). The interlocking member 92 is constituted by a wire, for example. When the wire (the interlocking member 92) is pulled toward the upper (Y1) side in the height direction Y of the user, the connected body 90 (the connection part 90A) is also moved toward the upper (Y1) side in the height direction Y of the user as shown in Fig. 7(B). Consequently, the first regulation side link 82 and the second regulation side link 84 assume the protruded posture to make the relative opening angle α smaller than 180 degrees, and thus the thigh side link 4 and the lower leg side link 6 are allowed to assume the knee bending posture.

When the pulled state of the wire (the interlocking member 92) is released, on the other hand, the connected body 90 (the connection part 90A) is pressed by the biasing part 88 to move toward the lower (Y2) side in the height direction Y of the user as shown in Fig. 7(A). Consequently, the first regulation side link 82 and the second regulation side link 84 assume the braking posture, and thus the thigh side link 4 and the lower leg side link 6 assume the knee extension posture.

### Movement 1 of Connected Body:

A configuration of the wire (the interlocking member 92) to move the connected body 90 (the connection part 90A) will be described next. The one end of the wire (the interlocking member 92) is connected to the waist side link 3 (the assist part 31), and the other end thereof is connected to the connected body 90. As shown in Figs. 9(A) and 9(B), the wire (the interlocking member 92) faces the thigh side link 4 on a more forward (Z2) side in the front-rear direction Z than the thigh side link 4 up to a midway point along a path from the waist side link 3 (the assist part 31) to the connected body 90.

An interlocking member guide part (or a wire guide part) 93 is provided from such a midway point to a point immediately before the connected body 90. The interlocking member guide part 93 guides the wire (the interlocking member 92) to the connected body 90 (the connection part 90A) while allowing the wire (the interlocking member 92) to pass across the thigh side link 4 (the thigh side link main body part 40) from the front (Z2) side to the rear (Z1) side in the front-rear direction Z of the thigh side link 4.

The wire (the interlocking member 92) is disposed in such a manner as to face the thigh side extended part 43 in the front-rear direction Z. Furthermore, a wire facing surface 46 of a leading end portion 44 of the thigh side extended part 43 is provided with a protruded part 45 having a hole through which the wire (the interlocking member 92) is passed. When the thigh side link 4 relatively swings with respect to the waist side link 3, the thigh side extended part 43 also relatively swings together with the thigh side link main body part 40.

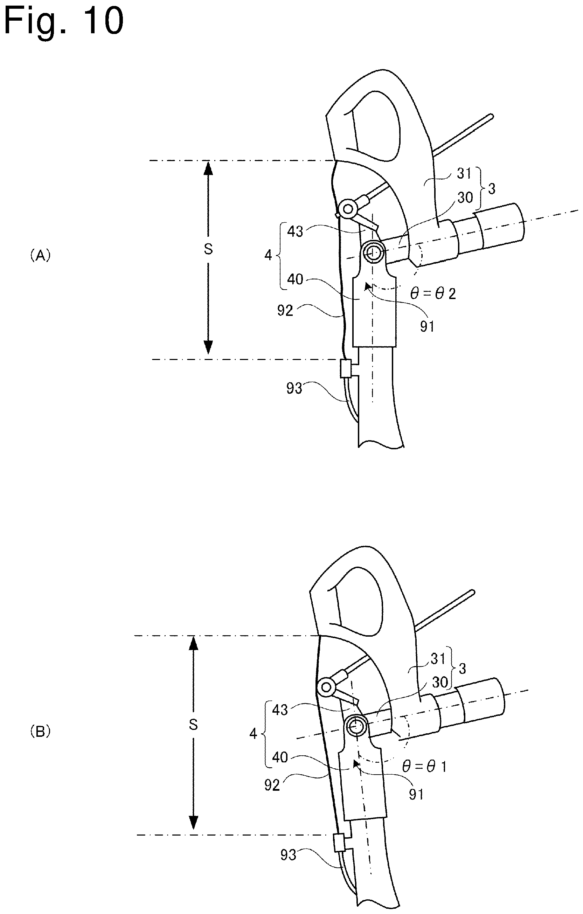

When the relative angle θ of the thigh side link 4 with respect to the waist side link 3 is equal to θ2 that is larger than the reference relative angle θ1, the wire (the interlocking member 92) is in a loose state as shown in Fig. 10(A). As seen in a midway segment S before the interlocking member guide part 93, the wire (the interlocking member 92) extends to the interlocking member guide part 93 while at least partially following a meandering path.

When the thigh side link 4 (the thigh side link main body part 40) in the state shown in Fig. 10(A) relatively swings with respect to the waist side link 3 toward the rear (Z1) side in the front-rear direction Z (counterclockwise in Fig. 10(B)) as shown in Fig. 10(B), the thigh side extended part 43 relatively swings toward the front (Z2) side in the front-rear direction Z (counterclockwise in Fig. 10(B)). Consequently, the leading end portion 44 of the thigh side extended part 43 moves in a direction away from the waist side link 3. The leading end portion 44 of the thigh side extended part 43 presses, while being in contact with, the wire (the interlocking member 92) to move the wire (the interlocking member 92) toward the front (Z2) side in the front-rear direction Z. From the standpoint of the thigh side link 4, the wire (the interlocking member 92) relatively moves in a direction away from the thigh side link 4. When the relative angle θ of the thigh side link 4 with respect to the waist side link 3 becomes equal to the reference relative angle θ1, the wire (the interlocking member 92) has a tense state.

As seen in the midway segment S before the interlocking member guide part 93, the wire (the interlocking member 92) extends to the interlocking member guide part 93 while following a path protruded toward the front (Z2) side in the front-rear direction Z. That is, as a result of the thigh side link 4 (the thigh side link main body part 40) relatively swinging with respect to the waist side link 3 toward the rear (Z1) side in the front-rear direction Z (counterclockwise in Fig. 10(B)), the path of the wire (the interlocking member 92) changes. At this time, the path length of the wire (the interlocking member 92) in the midway segment S becomes longer in the state shown in Fig. 10(B) than in the state shown in Fig. 10(A). The connected body 90 (the connection part 90A) is pulled up accordingly.

When the thigh side link 4 (the thigh side link main body part 40) in the state shown in Fig. 10(B) relatively swings with respect to the waist side link 3 toward the front (Z2) side in the front-rear direction Z (clockwise in Fig. 10(B)), the thigh side extended part 43 relatively swings toward the rear (Z1) side in the front-rear direction Z (clockwise in Fig. 10(B)). Consequently, the leading end portion 44 of the thigh side extended part 43 moves in a direction closer to the waist side link 3. At this time, the pressure on the wire (the interlocking member 92) is eliminated,, and thus the wire (the interlocking member 92) has a loose state. Consequently, the pull force on the connected body 90 is eliminated. At this time, the connected body 90 (the connection part 90A) is moved toward the lower side in the guide direction by the biasing part 88, and thus the first regulation side link 82 and the second regulation side link 84 return to positions to assume the braking posture.

Although the wire (the interlocking member 92) is held by the protruded part 45 in the present embodiment, the present invention is not limited thereto. The wire (the interlocking member 92) may be provided separately from the leading end portion 44 of the thigh side extended part 43 without being held.

Note that the present invention may not be limited to the above-described configuration as long as a configuration can change the path length of the wire (the interlocking member 92) in the midway segment S by coming into contact with the wire (the interlocking member 92) and thereby changing the path of the wire (the interlocking member 92) in the midway segment S along with the operation of the thigh side link 4 relatively swinging with respect to the waist side link 3. Another configuration will be described below.

### Movement 2 of Connected Body:

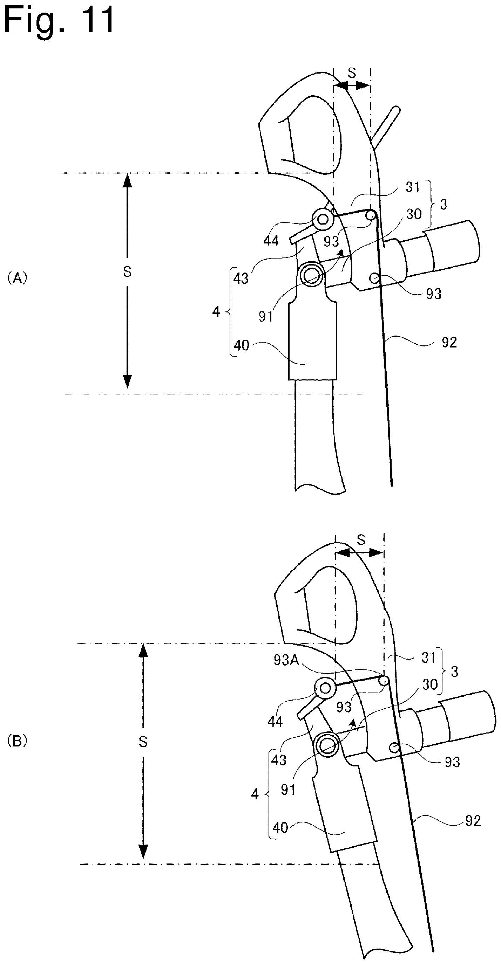

Another configuration of the wire (the interlocking member 92) to move the connected body 90 (the connection part 90A) will be described with reference to Fig. 11. As shown in Figs. 11(A) and 11(B), the wire (the interlocking member 92) may face the thigh side link 4 on a more rearward (Z1) side in the front-rear direction Z than the thigh side link 4, for example.

In this case, one end of the wire (the interlocking member 92) is connected to the thigh side extended part 43. The wire (the interlocking member 92) is guided to the connected body 90 via the interlocking member guide part 93. When the thigh side link 4 (the thigh side link main body part 40) relatively swings with respect to the waist side link 3 toward the rear (Z1) side in the front-rear direction Z (counterclockwise in Fig. 11(B)) as shown in Fig. 11(B), the thigh side extended part 43 relatively swings toward the front (Z2) side in the front-rear direction Z. Thus, the leading end portion 44 of the thigh side extended part 43 moves in a direction away from the waist side link 3. Consequently, the leading end portion 44 of the thigh side extended part 43 pulls the wire (the interlocking member 92). This changes the path of the wire (the interlocking member 92) in a midway segment S to make the path length of the wire (the interlocking member 92) longer. The connected body 90 (the connection part 90A) is pulled up accordingly. Note that the midway segment S herein refers to a segment extending from the leading end portion 44 of the thigh side extended part 43 to an entrance 93A of the interlocking member guide part 93.

When the thigh side link 4 (the thigh side link main body part 40) in the state shown in Fig. 11(B) relatively swings with respect to the waist side link 3 toward the front (Z2) side in the front-rear direction Z, on the other hand, the thigh side extended part 43 relatively swings toward the rear (Z1) side in the front-rear direction Z (clockwise in Fig. 11(B)). Thus, the leading end portion 44 of the thigh side extended part 43 moves in a direction closer to the waist side link 3. Consequently, the leading end portion 44 of the thigh side extended part 43 lowers the wire (the interlocking member 92). This changes the path of the wire (the interlocking member 92) in the midway segment S to make the path length of the wire (the interlocking member 92) shorter. The connected body 90 (the connection part 90A) is pulled down accordingly.

### Movement 3 of Connected Body:

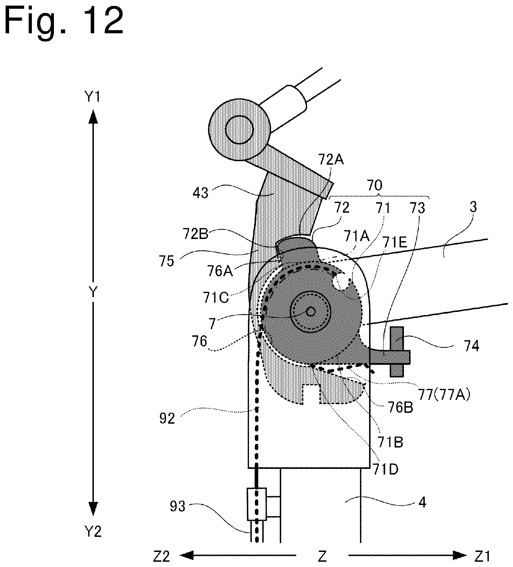

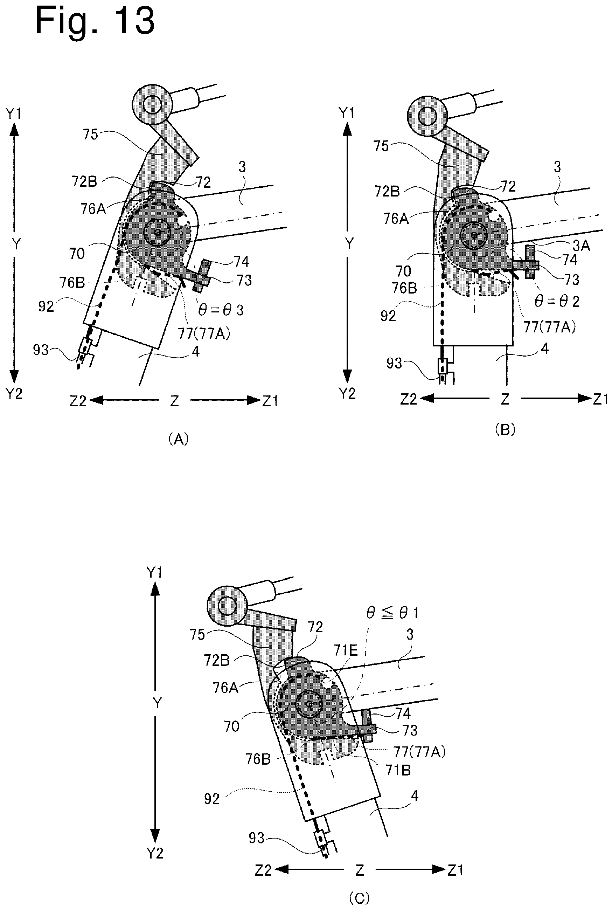

Still another configuration of the wire (the interlocking member 92) to move the connected body 90 (the connection part 90A) will be described with reference to Figs. 12 and 13. The moving mechanism 91 includes a lever member 70 and an engagement member 75. The lever member 70 rotates using the knee joint side swinging shaft 7 as an axis. As shown in Fig. 12, one end of the wire (the interlocking member 92) may be attached to the lever member 70. The lever member 70 as described above includes a disc-shaped main body part 71, a first protruded part 72, a second protruded part 73, and a third protruded part 74.

The first protruded part 72 is protruded in a radially outward direction starting from a predetermined region 71C on an outer peripheral surface of the main body part 71. The second protruded part 73 is protruded in a direction away from a predetermined region 71B on the outer peripheral surface of the main body part 71 starting from the region 71B. One example of the direction away from the region 71B is a tangent direction of an edge portion 71D in the region 71B, for example. The region 71B is disposed on the lower (Y2) side in the height direction Y than the waist side link 3. The region 71B is also disposed approximately opposite to the region 71C across the center of the main body part 71 (the same position as the knee joint side swinging shaft 7) as a reference. The second protruded part 73 is disposed on the lower (Y2) side in the height direction Y than the waist side link 3. The third protruded part 74 is protruded in a direction closer to the waist side link 3 starting from the second protruded part 73. The second protruded part 73 and the third protruded part 74 are disposed on the lower (Y2) side in the height direction Y than the waist side link 3. The first protruded part 72, the second protruded part 73, and the third protruded part 74 rotate integrally with the main body part 71 using the knee joint side swinging shaft 7 as an axis.

The main body part 71 is configured to be capable of relatively rotating about the knee joint side swinging shaft 7 with respect to the waist side link 3 and the thigh side link 4. The one end of the wire (the interlocking member 92) is fixed at the main body part 71. The main body part 71 also includes a main body side guide portion 71A for guiding the wire (the interlocking member 92) in a circumferential direction of the main body part 71 from a lever side fixed end 71E of the wire (the interlocking member 92). The main body side guide portion 71A is constituted, for example, by a passage extending in the circumferential direction of the main body part 71 inside the main body part 71.

The engagement member 75 including the thigh side extended part 43 is connected to the thigh side link 4. Thus, the engagement member 75 is configured to be capable of swinging integrally with the thigh side link 4 about the knee joint side swinging shaft 7. The engagement member 75 includes a surrounding surface 76 that extends along the circumferential direction of the lever member 70 from a leading end surface 72A of the first protruded part 72 in a protruded direction to a midway point in the second protruded part 73 so as to surround the outer peripheral surface of the lever member 70. The surrounding surface 76 also swings about the knee joint side swinging shaft 7.

The surrounding surface 76 includes a first protruded part side opposed surface 76A opposed to a vertically provided peripheral surface 72B of the first protruded part 72 in the circumferential direction of the main body part 71. The vertically provided peripheral surface 72B of the first protruded part 72 refers to a surface constituting the peripheral surface of the first protruded part 72 in the protruded direction and provided vertically from the main body part 71. Although the first protruded part side opposed surface 76A is in contact with the vertically provided peripheral surface 72B in the present embodiment, the present invention is not limited thereto. A spacer in contact with both of the first protruded part side opposed surface 76A and the vertically provided peripheral surface 72B may be provided between these surfaces. The surrounding surface 76 also includes a second protruded part side opposed surface 76B opposed to the second protruded part 73.

An elastic member 77 is provided between the second protruded part 73 and the second protruded part side opposed surface 76B. The elastic member 77 comes into contact with both of the second protruded part 73 and the second protruded part side opposed surface 76B. The elastic member 77 functions to press the first protruded part side opposed surface 76A against the vertically provided peripheral surface 72B of the first protruded part 72. That is, the elastic member 77 presses the engagement member 75 toward a clockwise side in the circumferential direction of the lever member 70 to maintain a state in which the engagement member 75 is in contact with the lever member 70. In that sense, it can be regarded that an engagement part to engage with the lever member 70 is constituted by the elastic member 77 and the engagement member 75. One example of the elastic member 77 is a plate spring 77A, for example, but the elastic member 77 may be any other elastic member. Assuming that the elastic member 77 is the plate spring 77A, operations of the moving mechanism 91 will be described below.

When the engagement member 75 swings, integrally with the thigh side link 4, toward the front (Z2) side in the front-rear direction Z about the knee joint side swinging shaft 7, the engagement member 75 rotates the lever member 70 clockwise via the first protruded part side opposed surface 76A in contact with the vertically provided peripheral surface 72B as shown in Fig. 13(A). Note that the state of the waist side link 3, the thigh side link 4, and the thigh side extended part 43 shown in Fig. 13(A) corresponds to the state of the walking assistance mechanism 1A shown in Fig. 4(A); the state of the waist side link 3, the thigh side link 4, and the thigh side extended part 43 shown in Fig. 13(B) corresponds to the state of the walking assistance mechanism 1A shown in Fig. 4(B); and the state of the waist side link 3, the thigh side link 4, and the thigh side extended part 43 shown in Fig. 13(C) corresponds to the state of the walking assistance mechanism 1A shown in Fig. 4(C).

When the engagement member 75 in the state shown in Fig. 13(A) swings toward the rear (Z1) side in the front-rear direction Z about the knee joint side swinging shaft 7, on the other hand, the engagement member 75 partway presses the lever member 70 via the first protruded part side opposed surface 76A in contact with the vertically provided peripheral surface 72B, swings in the same phase with the lever member 70, and rotates the lever member 70 clockwise as shown in Fig. 13(B). When the third protruded part 74 comes into contact with a lower surface 3A of the waist side link 3 from a lower side than the surface 3A as shown in Fig. 13(C), the rotation of the lever member 70 is regulated and only the engagement member 75 further swings. At this time, a phase difference is generated between the lever member 70 and the engagement member 75. The plate spring 77A becomes flat, thereby making the second protruded part side opposed surface 76B closer to the second protruded part 73. Note that the waist side link 3 functions as a rotation regulating part for regulating the rotation of the lever member 70. Note however that the rotation regulating part may be constituted by another member.

In this state, the vertically provided peripheral surface 72B and the first protruded part side opposed surface 76A become out of contact with each other as shown in Fig. 13(C), and start to separate from each other by a predetermined distance. The relative angle θ of the thigh side link 4 with respect to the waist side link 3 in the state in which the vertically provided peripheral surface 72B and the first protruded part side opposed surface 76A start to separate from each other becomes the reference relative angle θ1. At this time, the path of the wire (the interlocking member 92) in a segment from the lever side fixed end 71E of the wire (the interlocking member 92) to the interlocking member guide part 93 continues to change, and the path length becomes longer by an amount corresponding to the predetermined distance between the vertically provided peripheral surface 72B and the first protruded part side opposed surface 76A. As a result of this, the wire (the interlocking member 92) is pulled up. Consequently, the connected body 90 (the connection part 90A) is pulled up.

That is, the thigh side link 4 and the engagement member 75 swing toward the rear (Z1) side in the front-rear direction Z, and the lever member 70 rotates counterclockwise integrally with the engagement member 75 while having the same phase with the engagement member 75. Once the relative angle θ of the thigh side link 4 with respect to the waist side link 3 becomes equal to the reference relative angle θ1 (see Fig. 13(C)), the vertically provided peripheral surface 72B and the first protruded part side opposed surface 76A start to separate from each other. The lever member 70 has a phase difference with the engagement member 75 as shown in Fig. 13(C), and relatively rotates clockwise with respect to the engagement member 75. That is, from the standpoint of a system that operates together with the engagement member 75, the lever member 70 is turned on to start to wind the wire (the interlocking member 92) when the reference relative angle θ1 is reached. In that sense, the lever member 70, the engagement part (the engagement member 75 and the elastic member 77), and the waist side link 3 (the rotation regulating part) together constitute a winding mechanism. Consequently, the connected body 90 (the connection part 90A) is pulled up together with the wire (the interlocking member 92), and thus the posture of the first regulation side link 82 and the second regulation side link 84 is changed from the braking posture to the protruded posture. When the relative angle θ of the thigh side link 4 with respect to the waist side link 3 is larger than θ1 and the vertically provided peripheral surface 72B and the first protruded part side opposed surface 76A are in contact with each other as shown in Figs. 13(A) and 13(B), on the other hand, the lever member 70 rotates in the same phase with the engagement member 75. Thus, from the standpoint of the system that operates together with the engagement member 75, the lever member 70 keeps a switched-off state. Consequently, no wire (interlocking member 92) is wound by the lever member 70, and the first regulation side link 82 and the second regulation side link 84 maintain the braking posture.

### Walking Posture Ensuring Part 10:

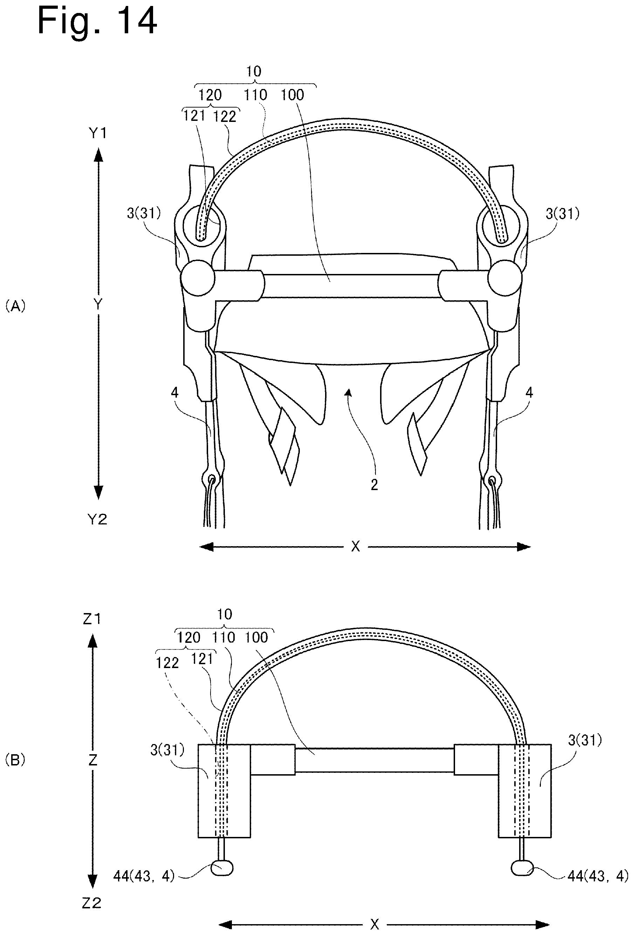

The walking posture ensuring part 10 will now be described with reference to Fig. 14. The walking posture ensuring part 10 is provided for ensuring the walking posture of the user. That is, the walking posture of the user refers to a posture in which when one leg swings toward a front side, the other leg swings toward a rear side.

As shown in Fig. 14(A), the walking posture ensuring part 10 includes the waist side connecting member 100, a bridging link 110, and a bridging link guide part 120. The waist side connecting member 100 is provided for integrally connecting the pair of waist side links 3 disposed on the right and left sides of the user. The waist side connecting member 100 is constituted, for example, by a cylindrical body having a cylindrical shape. Both ends of the cylindrical body are connected to the vicinities of the ends of the pair of waist side links 3 on the rear (Z1) side in the front-rear direction Z as shown in Fig. 14(B). That is, the waist side connecting member 100 is bridged between the pair of waist side links 3 so as to integrally connect the pair of waist side links 3.

As shown in Fig. 14(B), the bridging link 110 is bridged between the two walking assistance mechanisms and connected to the two thigh side links 4 while being guided by the bridging link guide part 120. The bridging link 110 is connected to the leading end portions 44 of the thigh side extended parts 43 in the thigh side links 4 to be able to swing about an extended part side swinging shaft 123, for example. Note that the extended part side swinging shaft 123 is a shaft extending in the width direction X.

The bridging link guide part 120 is provided for guiding the vicinities of the ends of the bridging link 110 in the front-rear direction Z. As shown in Fig. 14(B), the bridging link guide part 120 includes, for example, two lateral side guide portions 121 and a back side guide portion 122. The two lateral side guide portions 121 are constituted, for example, by passage holes passing completely through the right and left assist parts 31 in the front-rear direction. The back side guide portion 122 guides the bridging link 110 to the two lateral side guide portions 121 while surrounding the back side of the user in a curved manner, for example. The back side guide portion 122 is constituted, for example, by a curved tubular body.

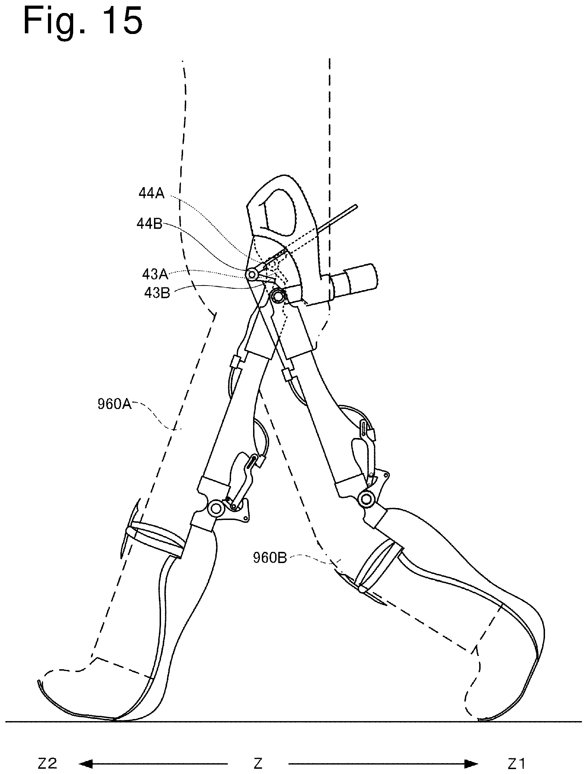

Positions, in the front-rear direction, of the leading end portions 44 of the right and left thigh side extended parts 43 when the user wearing the walking assistance device 1 is walking (see Fig. 15) will now be compared with each other, for example. When the positions, in the front-rear direction, of a leading end portion 44A of a thigh side extended part 43A corresponding to a leg 960A on the front (Z2) side in the front-rear direction Z and a leading end portion 44B of a thigh side extended part 43B corresponding to a leg 960B on the rear (Z1) side in the front-rear direction Z are compared with each other, the former is located on a more rearward (Z1) side in the front-rear direction Z than the latter as shown in Fig. 16(A). Subsequently, when the leg 960A is turned to be on the rear side and the leg 960B is turned to be on the front side, the leading end portion 44A of the thigh side extended part 43A is located on a more forward (Z2) side in the front-rear direction Z than the leading end portion 44B of the thigh side extended part 43B as shown in Fig. 16(B). That is, the moving direction of the one end of the bridging link 110 becomes opposite to the moving direction of the other end of the bridging link 110 along with the operations of the respective walking assistance mechanisms 1A in the front-rear direction Z. This can prevent both of the leg 960A and the leg 960B from moving in the same direction. Thus, the walking posture of the user can be ensured.

### Walking Action:

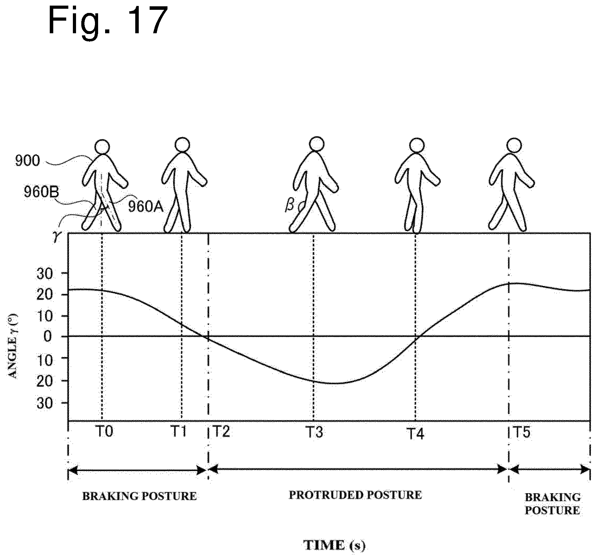

Walking action of the user 900 wearing the walking assistance device 1 will now be described with reference to Fig. 17. The user 900 at time T0 is positioning the leg 960A on a more forward side than the leg 960B. At this time, an angle γ of the thigh side link 4 with respect to the vertical direction is approximately +22 degrees. In such a state, the walking assistance mechanism (not shown) on the leg 960A side assumes the knee extension posture as a result of the relative swinging between the thigh side link 4 and the lower leg side link 6 being regulated by the knee extension posture maintenance mechanism 8.

The user 900 at time T1 is positioning the leg 960A on a more rearward side than at the time T0. At this time, the angle γ of the thigh side link 4 with respect to the vertical direction is approximately +7 degrees. Also in such a state, the walking assistance mechanism on the leg 960A side assumes the knee extension posture.

At time T2, the angle of the thigh side link 4 with respect to the vertical direction exceeds 0 degrees, and the leg 960A starts to move toward a more rearward side than the leg 960B. At this time, the thigh side link 4 forms the predetermined reference relative angle θ1 with respect to the waist side link 3. Thus, the walking assistance mechanism 1A on the leg 960A side assumes the knee bending posture as a result of the regulation of the knee extension posture maintenance mechanism 8 being released by the knee bending posture allowance mechanism 9.

At time T3, the angle of the thigh side link 4 with respect to the vertical direction becomes equal to approximately -20 degrees. Furthermore, the relative angle β between the thigh side link 4 and the lower leg side link 6 becomes smaller, and the degree of knee bending becomes larger. Up until time T4, the leg 960B of the user 900 moves toward the rear side and the leg 960A moves toward the front side. At the time T4, the angle of the thigh side link 4 with respect to the vertical direction exceeds 0 degrees, and the leg 960A starts to move toward a more forward side than the leg 960B. At this time, the thigh side link 4 forms the predetermined reference relative angle θ1 with respect to the waist side link 3, and thus the knee extension posture maintenance mechanism 8 starts to operate. That is, the first regulation side link 82 and the second regulation side link 84 start to change their positions to assume the braking posture by means of the biasing part 88. At time T5 when a predetermined amount of time has elapsed from the time T4, the first regulation side link 82 and the second regulation side link 84 achieve the braking posture. Thus, the walking assistance mechanism on the leg 960A side assumes the knee extension posture as a result of the regulation of the relative swinging between the thigh side link 4 and the lower leg side link 6.

Note that the angle γ of the thigh side link 4 with respect to the vertical direction at the time T5 is approximately +25 degrees and the relative angle θ of the thigh side link 4 with respect to the waist side link 3 is larger than θ1. Thus, even when the relative angle θ of the thigh side link 4 with respect to the waist side link 3 becomes larger than the predetermined reference relative angle θ1 in the course of achieving the knee extension posture from the knee bending posture as described above, the walking assistance mechanism on the leg 960A side keeps the knee bending posture until the first regulation side link 82 and the second regulation side link 84 achieve the braking posture.

As described above, the walking assistance device 1 can allow a knee on a leg of the user (pedestrian) of the walking assistance device 1 on the front side to maintain an extended state and allow a knee on a leg of the user (pedestrian) of the walking assistance device 1 on the rear side to be bent while walking.

### Second Embodiment:

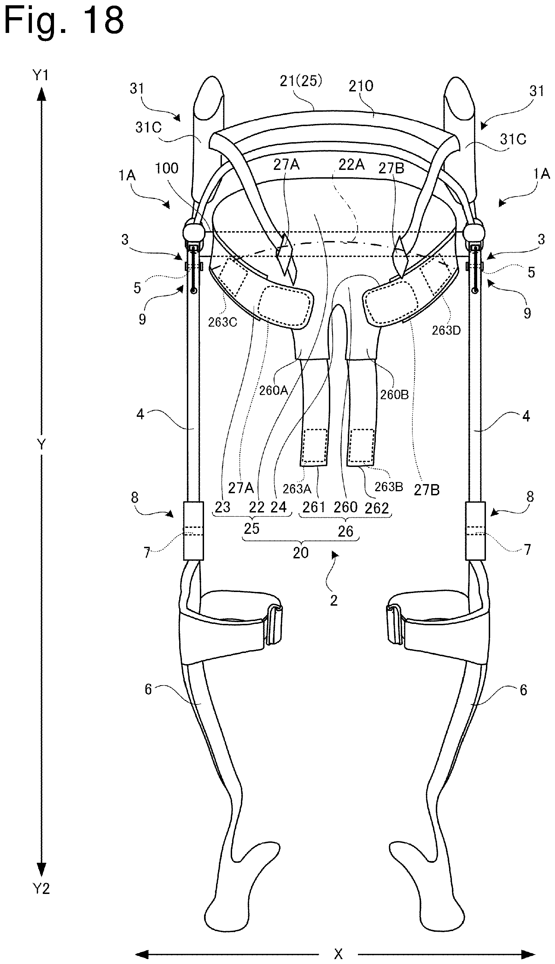

A walking assistance device 1 according to a second embodiment of the present invention will now be described with reference to Fig. 18. Features of the walking assistance device 1 in the present embodiment are similar to those of the walking assistance device 1 in the first embodiment. Among these features, however, a wearing part 2, a lateral pair of knee extension posture maintenance mechanisms 8, and a lateral pair of knee bending posture allowance mechanisms 9 have configurations different from those of the walking assistance device 1 in the first embodiment. Those configurations will be described below.

### Wearing Part:

The wearing part 2 will now be described with reference to Figs. 18 to 20. As shown in Fig. 18, the wearing part 2 includes, for example, a first belt part 20 and a second belt part 21. The first belt part 20 is arranged on the lower (Y2) side in the height direction Y than the second belt part 21. The first belt part 20 is provided at a position corresponding to approximately the waist portion of the human body. The second belt part 21 is provided at a position corresponding to the upper (Y1) side in the height direction Y than approximately the waist portion of the human body.

### First Belt Part:

The first belt part 20 includes a waist side belt part 25 and a crotch side belt part 26. The waist side belt part 25 is similar to that of the walking assistance device 1 according to the first embodiment of the present invention. Since the waist side belt part 25 has already been described, the description thereof will be omitted. In the waist side belt part 25 of the present embodiment, portions thereof are integrally formed as shown in Fig. 18, and thus the appearance thereof differs from that in the first embodiment. However, this is given by way of example, and different members may be connected to the waist side belt part 25. The same applies also to the waist side belt part 25 of the first embodiment.

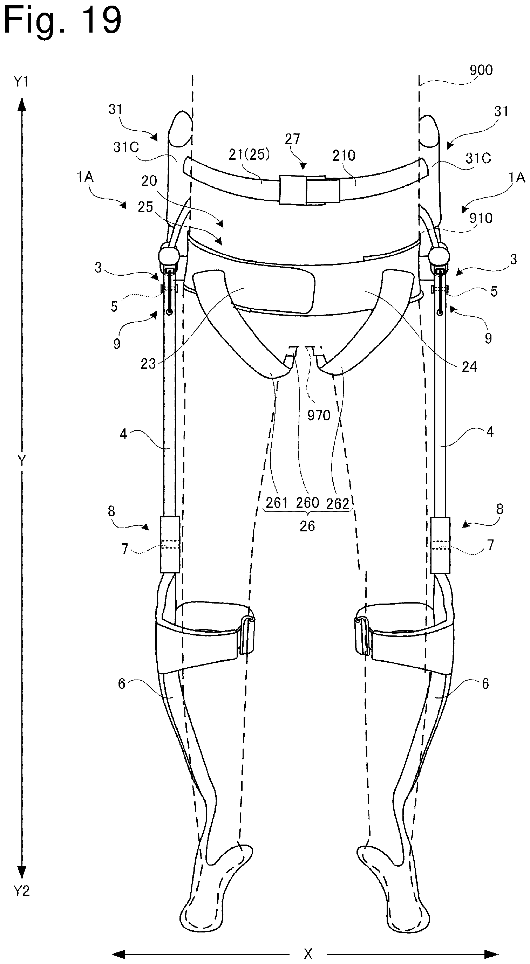

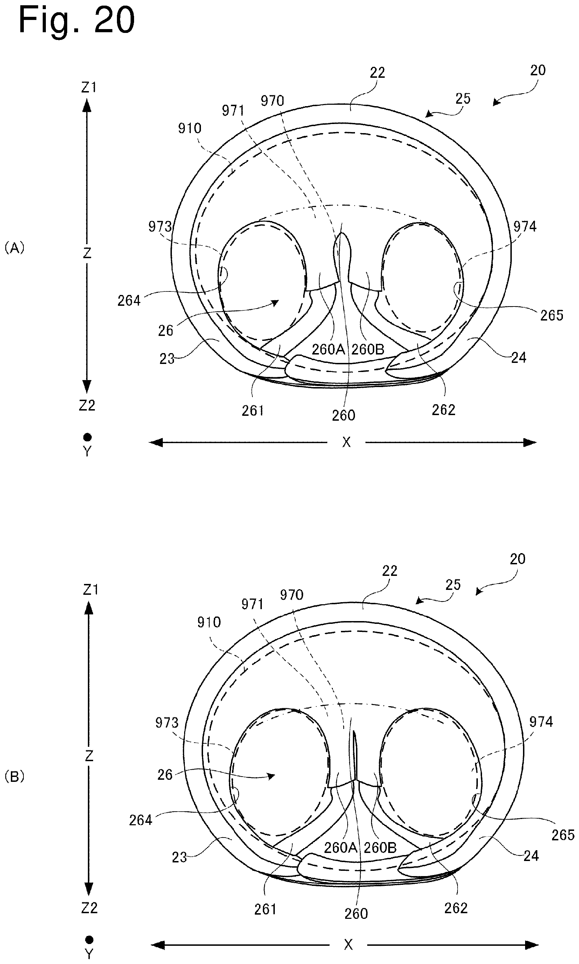

As shown in Fig. 19, the crotch side belt part 26 is provided for supporting a user 900 from the lower side in a crotch portion 970 of the user 900. As shown in Fig. 18, the crotch side belt part 26 includes, for example, a hip side support portion 260, a first crotch support portion 261, a second crotch support portion 262, and a connecting mechanism. As shown in Fig. 20(A), the hip side support portion 260 supports a hip 971 of the user 900 from the lower side in the height direction Y. As shown in Fig. 18, the hip side support portion 260 is a portion extended in the width direction of a lower back side belt portion 22 starting from a lower end portion 22A of the lower back side belt portion 22 in the width direction thereof. The hip side support portion 260 includes a first branch portion 260A and a second branch portion 260B bifurcated from a midway point of the hip side support portion 260 along the extending direction thereof.

The first crotch support portion 261 extends from a trailing end of the first branch portion 260A in the extending direction of the first branch portion 260A. The second crotch support portion 262 extends from a trailing end of the second branch portion 260B in the extending direction of the second branch portion 260B.

The connecting mechanism is provided for connecting the first crotch support portion 261 and the second crotch support portion 262 to a first belly side belt portion 23 and a second belly side belt portion 24, respectively. The connecting mechanism includes a third connection part 263A, a fourth connection part 263B, a fifth connection part 263C, and a sixth connection part 263D as shown in Fig. 18. The third connection part 263A and the fourth connection part 263B are provided in the vicinities of leading ends of the first crotch support portion 261 and the second crotch support portion 262, respectively. The fifth connection part 263C is provided on a front surface of the first belly side belt portion 23 opposite to the side abutting against the user 900. The sixth connection part 263D is provided on a front surface of the second belly side belt portion 24 opposite to the side abutting against the user 900.

The third connection part 263A and the fifth connection part 263C have structures capable of detachably connecting to each other. The fourth connection part 263B and the sixth connection part 263D have structures capable of detachably connecting to each other. The first crotch support portion 261 is connected to the first belly side belt portion 23 by connecting the third connection part 263A and the fifth connection part 263C together. The second crotch support portion 262 is connected to the second belly side belt portion 24 by connecting the fourth connection part 263B and the sixth connection part 263D together.

Examples of the above-described connecting mechanism include a connecting mechanism using Magic Tape (registered trademark), a connecting mechanism using a button, and an inserting part and a buckle to be locked with, and connected to, the inserting part when the inserting part is inserted thereinto. The third connection part 263A and the fifth connection part 263C as well as the fourth connection part 263B and the sixth connection part 263D may be those corresponding to the above by way of example.

### Wearing of First Belt Part:

When the user 900 wears the first belt part 20, the waist side belt part 25 surrounds a waist portion 910 of the user 900 and constricts the waist portion 910 as shown in Figs. 19 and 20(A). The crotch side belt part 26 supports the user 900 from the lower side in the crotch portion 970 of the user 900.

At this time, the hip side support portion 260 abuts against the hip 971 of the user 900 as shown in Figs. 20(A) and 20(B). The first branch portion 260A and the second branch portion 260B, together with the first crotch support portion 261 and the second crotch support portion 262, pass through the crotch portion 970 of the user 900. Once the first crotch support portion 261 is connected to the first belly side belt portion 23 via the connecting mechanism, the hip side support portion 260, the first crotch support portion 261, the first belly side belt portion 23, and part of the lower back side belt portion 22 are connected together in a ring shape. Consequently, inner peripheral edges of the hip side support portion 260, the first crotch support portion 261, the first belly side belt portion 23, and the part of the lower back side belt portion 22 are continuous with one another in a ring shape to form a hole 264. The hole 264 serves as a right leg insertion hole through which a right leg 973 of the user 900 is passed. Similarly, once the second crotch support portion 262 is connected to the second belly side belt portion 24 via the connecting mechanism, the hip side support portion 260, the second crotch support portion 262, the second belly side belt portion 24, and part of the lower back side belt portion 22 are also connected together in a ring shape. Consequently, inner peripheral edges of the hip side support portion 260, the second crotch support portion 262, the second belly side belt portion 24, and the part of the lower back side belt portion 22 are continuous with one another in a ring shape to form a hole 265. The hole 265 serves as a left leg insertion hole through which a left leg 974 of the user 900 is passed.

The waist side belt part 25 is then fixed by being attached to a member therearound (e.g., a waist side connecting member 100 or a waist side link 3) in such a manner that the waist side belt part 25 is positioned around the waist portion 910 of the user 900 for the user 900 wearing the walking assistance device 1. The state in which the user 900 is wearing the waist side belt part 25 around the waist portion 910 is defined as a normal wearing state. If the fastening of the waist side belt part 25 around the waist portion 910 of the user 900 is loose, the waist side belt part 25, with absence of the crotch side belt part 26, may possibly be displaced toward the upper side than the waist portion 910, thus failing to maintain the normal wearing state for the user 900 having difficulty in maintaining a standing posture. In the present embodiment, however, the user 900 is supported from the lower side by the crotch side belt part 26 in the crotch portion 970 of the user 900. Thus, even when the fastening of the waist side belt part 25 around the waist portion 910 of the user 900 is loose, there is no possibility that the waist side belt part 25 is displaced toward the upper side than the waist portion 910. Thus, the user 900 can maintain the normal wearing state.

As shown in Fig. 20(B), the hole 264 serving as the right leg insertion hole and the hole 265 serving as the left leg insertion hole can be expanded in their sizes as a result of the first branch portion 260A and the second branch portion 260B being deformed so as to come closer to each other. Even when a person with large thighs wears the crotch side belt part 26, for example, the hole 264 and the hole 265 can be expanded according to the thicknesses of the thighs. Thus, wearing is easy to perform.

If the hip side support portion 260 is unbranched, the width of the hip side support portion 260 needs to be reduced in order to expand the hole 264 and the hole 265. This, however, creates crinkles in the hip side support portion 260. When the hip side support portion 260 becomes crinkled, the surface of the hip side support portion 260 abutting against the crotch portion 970 of the user 900 becomes wavy. This may cause discomfort to the user 900. If the hip side support portion 260 is branched as in the present embodiment, no surfaces of the first branch portion 260A and the second branch portion 260B abutting against the crotch portion 970 of the user 900 become wavy, and thus the user 900 feels no discomfort.

### Second Belt Part:

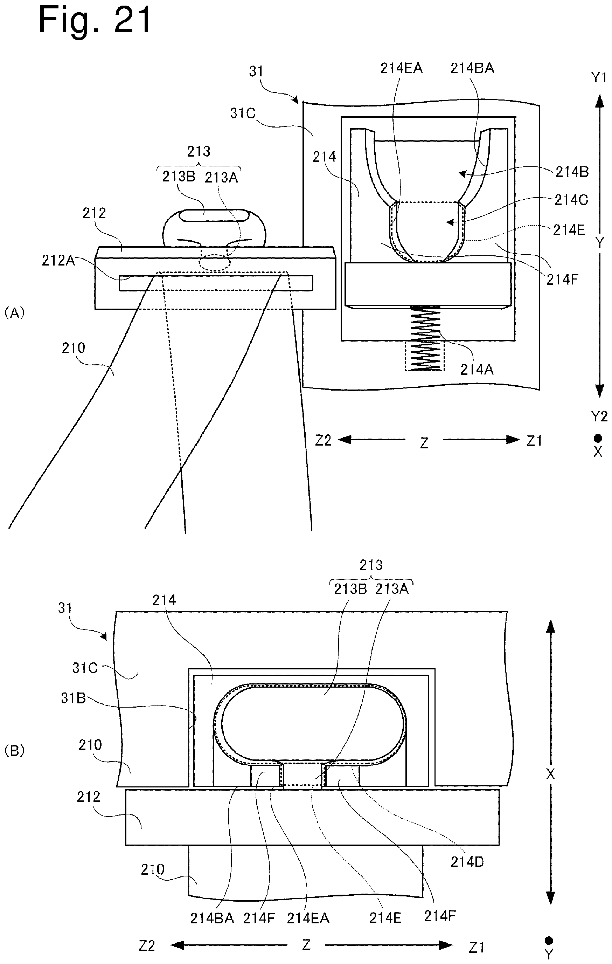

Referring to Figs. 18 and 21, the second belt part 21 in the present embodiment may be constituted by a waist side belt part 25 as shown in Fig. 18. Although the second belt part 21 in the present embodiment differs from the waist side belt part 25 in the first belt part 20 in its width and aspect of a connecting mechanism, the present invention is not limited thereto. The second belt part 21 in the present embodiment may be implemented in a manner similar to the waist side belt part 25 in the first belt part 20. Moreover, the second belt part 21 in the present embodiment is arranged on the upper (Y1) side in the height direction Y than the first belt part 20. Specifically, the second belt part 21 is wound on the upper side than the waist portion 910 of the user 900.

Note however that a lower back side belt portion 22, a first belly side belt portion 23, and a second belly side belt portion 24 of the waist side belt part 25 in the second belt part 21 are constituted by a strip-shaped body 210 formed integrally in a strip shape. The strip-shaped body 210 has a length encompassing the waist portion 910. The strip-shaped body 210 also has flexibility. Although the strip-shaped body 210 may be a flat belt woven in a sheet shape with a synthetic resin such as nylon, for example, the present invention is not limited thereto. Any other strip-shaped body 210 made of a material having flexibility may be employed. Since a connecting mechanism 27 in the second belt part 21 is similar to that in the first embodiment, the description thereof will be omitted.

The second belt part 21 also includes a length adjustment mechanism (not shown), a strip-shaped body holding part 212, and a seventh connection part 213. The length adjustment mechanism is provided for adjusting the length of the strip-shaped body 210 for constricting the user 900. Specifically, the length adjustment mechanism relatively moves a first connection part 27A and/or a second connection part 27B that constitute the connecting mechanism 27 along the strip-shaped body 210 as with the length adjustment mechanism in the first embodiment. Configuring the first connection part 27A or the second connection part 27B in this manner substantially enables the length of the strip-shaped body 210 worn by the user 900 to be adjusted. Note that the length adjustment part is not limited to the configuration described above, but may have any other configuration as with the length adjustment mechanism in the first embodiment.

As shown in Fig. 21(A), the strip-shaped body holding part 212 has a generally rectangular solid shape and includes a hole 212A through which the strip-shaped body 210 is passed. The hole 212A passes completely through the strip-shaped body holding part 212. Once the strip-shaped body 210 is passed through the hole 212A, the strip-shaped body 210 gets caught in, and is held by, the strip-shaped body holding part 212.

The seventh connection part 213 is provided in the strip-shaped body holding part 212. As shown in Fig. 21(A), the seventh connection part 213 includes, for example, a protruded portion 213A protruded from an outer peripheral surface of the strip-shaped body holding part 212, and a head portion 213B provided at a leading edge of the protruded portion 213A. The head portion 213B has a width larger than that of the protruded portion 213A.

On the other hand, an eighth connection part 214 that constitutes a connecting mechanism in cooperation with the seventh connection part 213 is provided in an upper portion 31C of an assist part 31 extending toward the upper (Y1) side in the height direction Y than the waist side link 3 as shown in Fig. 21(A). As shown in Fig. 21(B), the eighth connection part 214 is provided in an assist part side depressed portion 31B depressed in the width direction X starting from the side facing the user 900 in the upper portion 31C of the assist part 31. As shown in Fig. 21(A), the eighth connection part 214 includes, for example, a spring portion 214A, an insertion side depressed portion 214B, and a hooking portion 214C to hook the head portion 213B.

The spring portion 214A is provided in a lower end portion of the eighth connection part 214 so as to be capable of moving the eighth connection part 214 up and down in the height direction Y in the assist part side depressed portion 31B. The insertion side depressed portion 214B, which is a depressed portion provided in the eighth connection part 214, is opened to the side facing the user 900 through an opening 214BA and depressed in the width direction X. The insertion side depressed portion 214B has a size into which the seventh connection part 213 can be inserted in the width direction X through the opening 214BA.

The hooking portion 214C, which is a depressed portion provided in the eighth connection part 214, is continuous with the insertion side depressed portion 214B and depressed toward the lower (Y2) side in the height direction Y. As shown in Fig. 21(B), the hooking portion 214C includes, for example, a head portion insertion region 214D into which the head portion 213B can be inserted, and a protruded portion insertion region 214E into which the protruded portion 213A can be inserted. The head portion insertion region 214D and the protruded portion insertion region 214E are continuous with each other in this order in the width direction X from the outer side toward the inner side. The head portion insertion region 214D has a width larger than that of the protruded portion insertion region 214E. Thus, eighth connection part side walls 214F that constitute part of the eighth connection part 214 are provided on both sides of the protruded portion insertion region 214E in the width direction (the front-rear direction Z). The head portion insertion region 214D has a width (a length in the front-rear direction Z) larger than that of the head portion 213B. The protruded portion insertion region 214E has a width larger than that of the protruded portion 213A. The protruded portion insertion region 214E is opened to the side facing the user 900 through an opening 214EA.

By lowering the seventh connection part 213 inserted into the insertion side depressed portion 214B toward the lower (Y2) side in the height direction Y, the protruded portion 213A is inserted into the protruded portion insertion region 214E and the head portion 213B is inserted into the head portion insertion region 214D. Even when the strip-shaped body holding part 212 is pulled toward the inner side in the width direction X, the head portion 213B comes into contact with, and thereby caught by, the eighth connection part side walls 214F. Thus, the seventh connection part 213 is prevented from coming off from the eighth connection part 214. By connecting the seventh connection part 213 with the eighth connection part 214, the strip-shaped body 210 is positioned on the upper side than the waist portion 910 of the user 900.

### Knee Extension Posture Maintenance Mechanism:

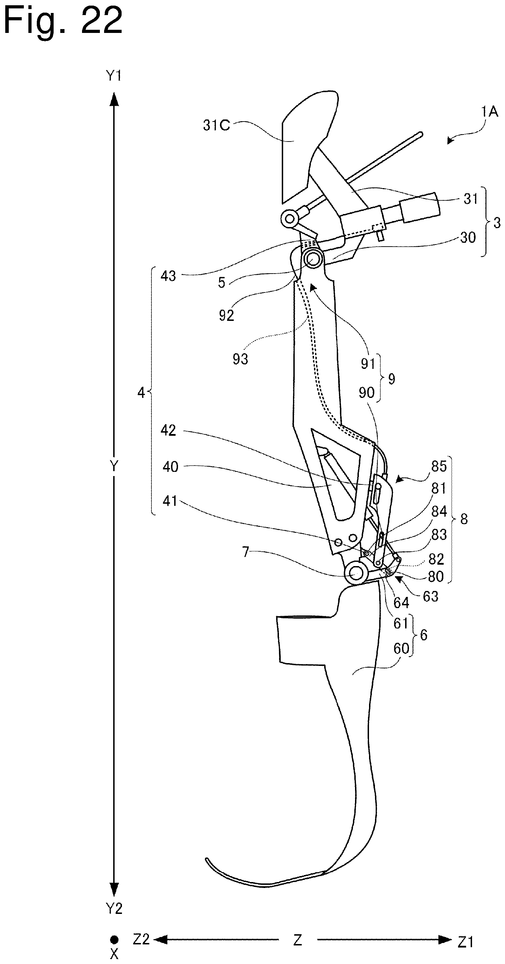

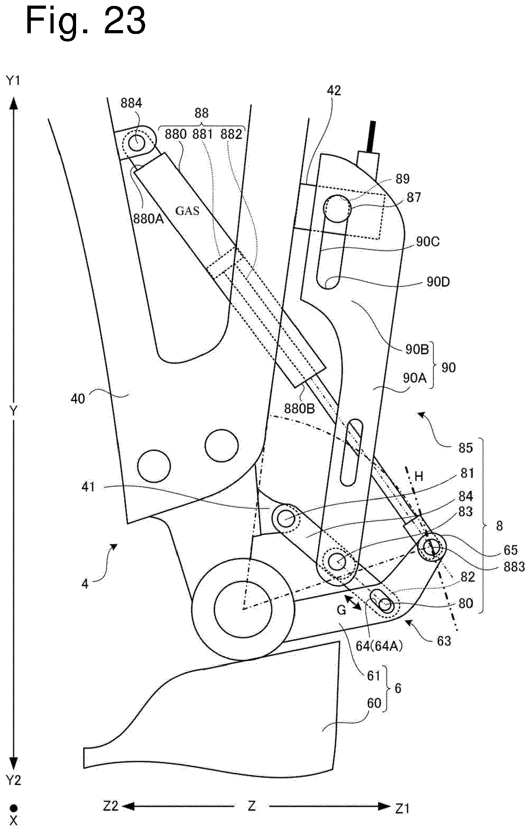

The knee extension posture maintenance mechanism 8 of the present embodiment will be described below with reference to Figs. 22 to 26. As shown in Figs. 22 and 23, the knee extension posture maintenance mechanism 8 of the present embodiment includes a lower leg side regulation shaft 80, a thigh side regulation shaft 81, a first regulation side link 82, an intermediate regulation shaft 83, a second regulation side link 84, and a stopper mechanism 85 as with the knee extension posture maintenance mechanism 8 of the first embodiment. Also in the present embodiment, a four bar linkage is formed by connecting a lower leg side protruded part 61 (a lower leg side link 6), the first regulation side link 82, the second regulation side link 84, and a first thigh side protruded part 41 (a thigh side link 4) to one another by means of a knee joint side swinging shaft 7, the lower leg side regulation shaft 80, the thigh side regulation shaft 81, and the intermediate regulation shaft 83 to be able to relatively swing. Since connection between the lower leg side protruded part 61 and the lower leg side regulation shaft 80, and the stopper mechanism 85 in the knee extension posture maintenance mechanism 8 of the present embodiment are implemented in a manner different from the knee extension posture maintenance mechanism 8 in the first embodiment, those respects will be described below.

### Lower Leg Side Protruded Part and Lower Leg Side Regulation Shaft:

As shown in Figs. 22 and 23, the lower leg side protruded part 61 includes a lower leg side regulation shaft guide part 64 at an intermediate position 63 in the front-rear direction Z. The lower leg side regulation shaft guide part 64 guides the lower leg side regulation shaft 80 in a guide direction G. The guide direction G refers to a direction tilted so as to come closer to the thigh side link 4 (or approach the front (Z2) side from the rear (Z1) side in the front-rear direction Z) as approaching the upper (Y1) side from the lower (Y2) side in the height direction Y when the lower leg side link 6 assumes a knee extension posture with respect to the thigh side link 4. The guide direction G in Fig. 23 is a direction along a straight line (see a dashed-dotted line in Fig. 23) connecting between the center of the lower leg side regulation shaft 80 and the center of the intermediate regulation shaft 83 (or the center of the thigh side regulation shaft 81) when the first regulation side link 82 and the second regulation side link 84 are assuming a braking posture. Note that the guide direction G is a direction along a straight line connecting between the center of the lower leg side regulation shaft 80 and the center of the intermediate regulation shaft 83 or the center of the thigh side regulation shaft 81 even when a relative opening angle α, which represents a degree of opening between the first regulation side link 82 and the second regulation side link 84, is larger than or equal to 180 degrees (see Fig. 6(A)), for example.

As shown in Fig. 23, the lower leg side regulation shaft guide part 64 in the present embodiment is constituted by a guide groove 64A. As shown in Fig. 23, the guide groove 64A is provided at the intermediate position 63 in the front-rear direction Z, and has a rounded rectangular shape as viewed in the width direction X, for example. That is, the guide groove 64A has a rounded rectangular shape in a Y-Z plane. The guide groove 64A passes completely through the lower leg side protruded part 61 in the width direction X in a rounded rectangular shape to have a rounded rectangle columnar shape. Note that the guide groove 64A may be constituted by a depressed portion depressed in the width direction X in the lower leg side protruded part 61 without passing completely through the lower leg side protruded part 61 in the width direction X. The width of the guide groove 64 perpendicular to the guide direction G is slightly larger than the diameter of the lower leg side regulation shaft 80. This allows the lower leg side regulation shaft 80 to be inserted into the guide groove 64 and held, while maintaining a posture extending in the width direction X, by the guide groove 64 in a manner capable of moving in the guide direction.

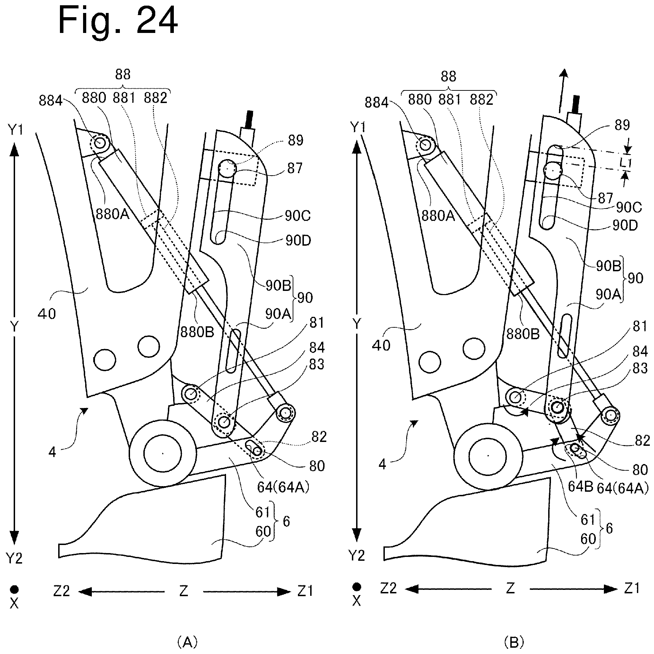

As shown in Fig. 24(B), when a connected body 90 moves toward an upper side in a guide direction of the connected body 90 (the upper (Y1) side in the height direction Y) by a distance L1 together with the intermediate regulation shaft 83, the first regulation side link 82 and the second regulation side link 84 of the four bar linkage are pulled in the same direction. At this time, the lower leg side regulation shaft 80 is pulled by the first regulation side link 82, thereby moving, along the guide groove 64, to an end portion 64B on the side closer to the thigh side link 4 in the guide direction. At the same time, the first regulation side link 82 swings toward the rear (Z1) side in the front-rear direction Z (clockwise in Fig. 24) using the lower leg side regulation shaft 80 as an axis. The second regulation side link 84 swings toward the front (Z2) side in the front-rear direction Z (counterclockwise in Fig. 24) using the thigh side regulation shaft 81 as an axis. Consequently, the first regulation side link 82 and the second regulation side link 84 assume a protruded posture protruded toward the upper (Y1) side in the height direction Y. Note however that no lower leg side link 6 at this time swings. That is, when the moving distance of the connected body 90 is smaller than or equal to L1, only the first regulation side link 82 and the second regulation side link 84 of the four bar linkage swing, and no lower leg side link 6 swings. As described above, the four bar linkage in the present embodiment has an allowance in the operations of the first regulation side link 82 and the second regulation side link 84 due to the guide groove 64. Thus, even when the connected body 90 is caused to move a little under a situation unintended by the user 900 and the first regulation side link 82 and the second regulation side link 84 are thereby pulled toward the upper side, no knee bending posture is assumed immediately. Such an allowance in the operations of the first regulation side link 82 and the second regulation side link 84 can be adjusted in accordance with the length of the guide groove 64A in the guide direction G, and the degree of tilt of the guide direction G with respect to the height direction Y.

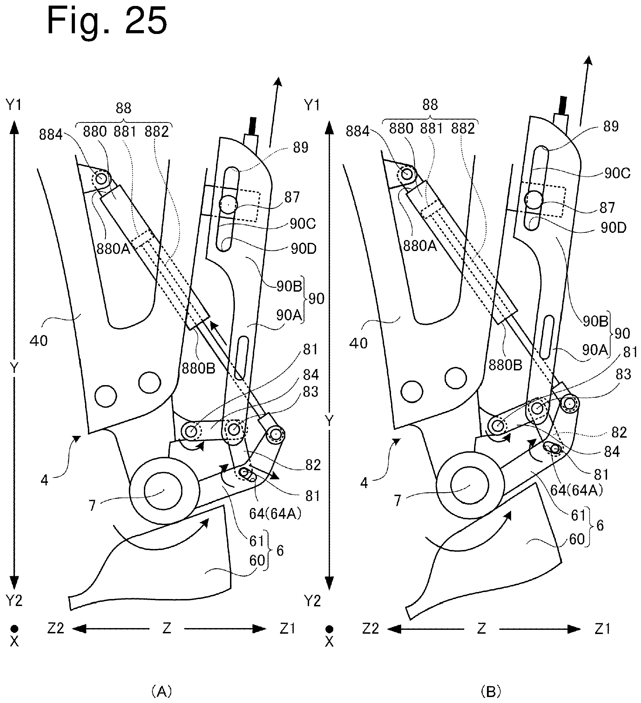

When the moving distance of the connected body 90 is larger than L1, the first regulation side link 82 and the second regulation side link 84 are further pulled in the same direction, and the first regulation side link 82 and the second regulation side link 84 swing. At this time, the lower leg side link 6 starts to swing toward the rear (Z1) side in the front-rear direction Z (counterclockwise in Fig. 25) using the knee joint side swinging shaft 7 as an axis as shown in Fig. 25(A). That is, each part of the four bar linkage starts to operate along with the operations of the other links.

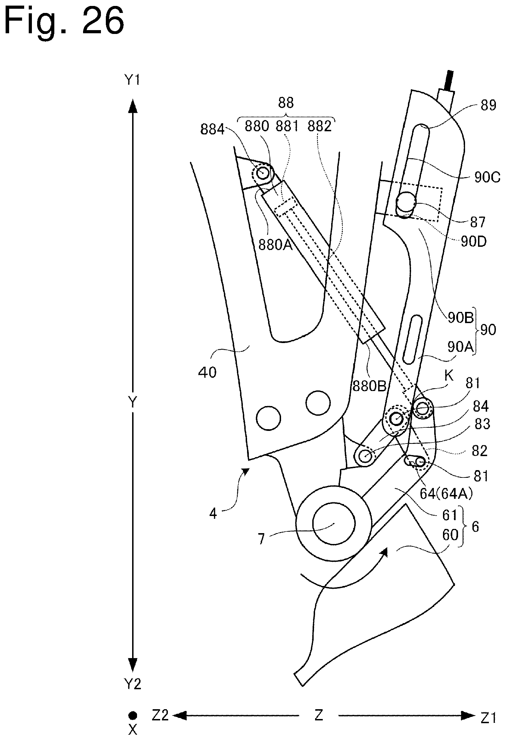

The lower leg side protruded part 61 changes its posture together with the guide groove 64. Thus, when the first regulation side link 82 and the second regulation side link 84 are pulled in the same direction, the lower leg side regulation shaft 80 may move, for example, in a direction away from the thigh side link 4 along the guide groove 64 as shown in Fig. 25(A) to be positioned at an end portion of the guide groove 64 distal to the thigh side link 4 as shown in Fig. 25(B). That is, the lower leg side regulation shaft 80 changes its position in the guide groove 64 in accordance with the postures of the lower leg side protruded part 61 and the first regulation side link 82. The lower leg side link 6 swings toward the rear (Z1) side in the front-rear direction Z (counterclockwise in Fig. 25) until the lower leg side protruded part 61 comes into contact with the connected body 90 at a contact point K as shown in Fig. 26, for example.

Note that a portion corresponding to a region where the connected body 90 and the lower leg side protruded part 61 interfere with each other (a region including the contact point K) when the lower leg side protruded part 61 further swings toward the rear (Z1) side in the front-rear direction Z (counterclockwise in Fig. 25) may be eliminated from the connected body 90 to make such a portion as a passage space for the lower leg side protruded part 61 to pass through so that the lower leg side protruded part 61 can pass through the connected body 90. In this case, the lower leg side protruded part 61 can swing toward a more rearward (Z1) side in the front-rear direction Z (counterclockwise in Fig. 25). Note however that a limit position up to which the lower leg side link 6 can swing can be, also in this case, associated with contact between the lower leg side protruded part 61 and the second regulation side link 84, or a maximum shrink position of a biasing part 88 to be described later, a limit position of the connected body 90 for upward movement (a lower end portion 90D of a through hole 90C to be in contact with a guide part 87), etc., for example.

### Stopper Mechanism:

The stopper mechanism 85 in the present embodiment will now be described with reference to Fig. 23. The stopper mechanism 85 in the present embodiment includes the connected body 90, the guide part 87, the biasing part 88, and a moving range limiting part 89 as with the stopper mechanism 85 in the first embodiment. Since the connected body 90, the guide part 87, and the moving range limiting part 89 in the present embodiment are similar to those in the first embodiment and have already been described, the description thereof will be omitted.

The biasing part 88 in the first embodiment biases the connected body 90 (the connection part 90A) so as to press the connected body 90 (the connection part 90A) in the direction making the relative opening angle α between the first regulation side link 82 and the second regulation side link 84 larger. The biasing part 88 in the present embodiment, on the other hand, biases the lower leg side protruded part 61 (the lower leg side link 6) so as to press the lower leg side protruded part 61 (the lower leg side link 6) to a knee extension position corresponding to the knee extension posture as shown in Fig. 23. That is, when the lower leg side protruded part 61 (the lower leg side link 6) swings toward the rear (Z1) side in the front-rear direction Z (counterclockwise in Fig. 25), the biasing part 88 presses the lower leg side protruded part 61 (the lower leg side link 6) so that the lower leg side protruded part 61 (the lower leg side link 6) swings toward the front (Z2) side in the front-rear direction Z (clockwise in Fig. 25).

The biasing part 88 includes, for example, a cylinder 880, a piston 881, and a rod 882. A gas (e.g., a nitrogen gas) is filled in the cylinder 880. The piston 881 is provided in the cylinder 880 so as to be movable in the axial direction of the cylinder 880. When the lower leg side protruded part 61 (the lower leg side link 6) is at the knee extension position with respect to the thigh side link 4, the piston 881 is at equilibrium and thus at rest, or pressed toward a leading end 880B of the cylinder 880 in the axial direction of the cylinder 880 by the gas. When the piston 881 moves toward a base end 880A of the cylinder 880 in the axial direction of the cylinder 880, the gas is compressed by the piston 881. Thus, the piston 881 receives a reaction force in a direction opposite to the moving direction from the compressed gas accordingly.

The rod 882 is connected to the piston 881 in such a manner that the axial direction of the rod 882 coincides with the axial direction of the cylinder 880 (or the piston 881). Part of the rod 882 is housed in the cylinder 880, and the remaining part of the rod 882 is projected outwardly from the leading end 880B side of the cylinder 880. The rod 882 is connected to the piston 881 so as to be movable in the axial direction of the cylinder 880 together with the piston 881.

A leading end of the rod 882 is connected to a leading end 65 of the lower leg side protruded part 61 via a first biasing side shaft 883. The first biasing side shaft 883 is a shaft extending in the width direction X. Thus, the rod 882 can relatively swing with respect to the lower leg side protruded part 61 (the lower leg side link 6) using the first biasing side shaft 883 as an axis. Note that the axial direction of the rod 882 becomes, for example, parallel to a direction tilted toward a more forward (Z2) side in the front-rear direction Z (clockwise about the first biasing side shaft 883 in Fig. 23) than a tangent direction H of an orbit of the center of the first biasing side shaft 883 when the lower leg side protruded part 61 (the lower leg side link 6) is at the knee extension position with respect to the thigh side link 4.

The base end 880A of the cylinder 880 is connected to the thigh side link 4 (a thigh side link main body part 40) via a second biasing side shaft 884. The second biasing side shaft 884 is a shaft extending in the width direction X. Thus, the cylinder 880 can relatively swing with respect to the thigh side link 4 using the second biasing side shaft 884 as an axis.

### Operations of Stopper Mechanism:

The operations of the stopper mechanism 85 in the present embodiment will now be described with reference to Figs. 23 to 26. When the knee extension posture (see Fig. 24(B)) in which the lower leg side protruded part 61 (the lower leg side link 6) is at the knee extension position with respect to the thigh side link 4 is turned into the knee bending posture (see Fig. 25(A)) in which the lower leg side protruded part 61 (the lower leg side link 6) is at a knee bending position with respect to the thigh side link 4, the rod 882 is pressed toward the base end 880A side of the cylinder 880 in the axial direction of the cylinder 880 by the lower leg side protruded part 61 (the lower leg side link 6), thereby moving toward the inner side of the cylinder 880. At this time, the piston 881 moves in the same direction together with the rod 882, thereby compressing the gas. As the degree of the knee bending posture further increases, the rod 882 further moves in the same direction together with the piston 881 (see Figs. 25(B) and 26), compresses the gas, and receives a large reaction force in the opposite direction from the gas.

When the knee bending posture returns to the knee extension posture, the piston 881 moves, together with the rod 882, toward the leading end 880B side of the cylinder 880 in the axial direction of the cylinder 880 by receiving the reaction force from the gas. The rod 882 presses the lower leg side protruded part 61 (the lower leg side link 6) to the knee extension position.

### Knee Bending Posture Allowance Mechanism:

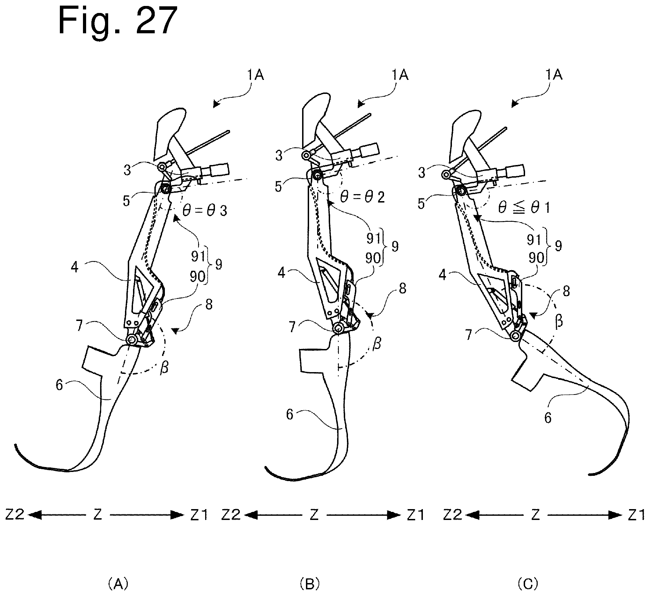

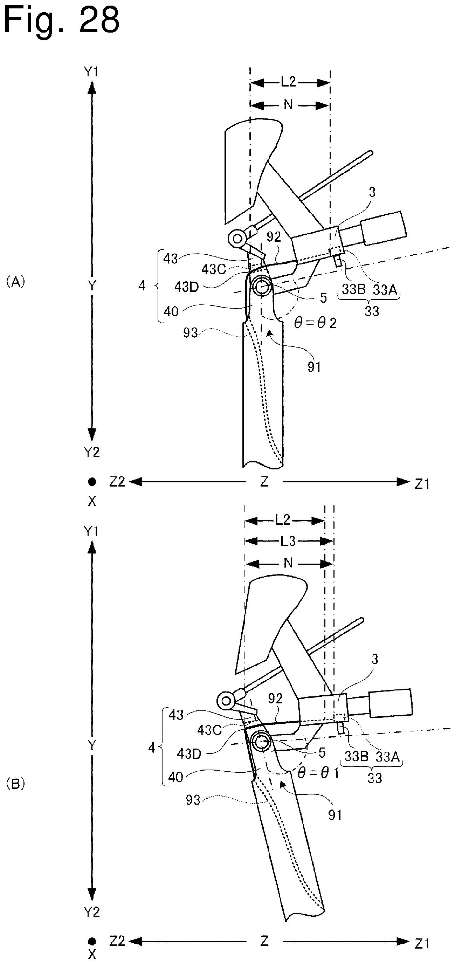

The knee bending posture allowance mechanism 9 in the present embodiment will be described below with reference to Figs. 27 and 28. Also in the walking assistance device 1 of the present embodiment, the knee bending posture allowance mechanism 9 allows for the knee bending posture in which a relative angle β between the thigh side link 4 and the lower leg side link 6 becomes smaller depending on the value of a relative angle θ formed by the thigh side link 4 with respect to the waist side link 3 as shown in Figs. 27(A) to 27(C). Although the knee bending posture allowance mechanism 9 includes the connected body 90 (the connection part 90A) and a moving mechanism 91 as with the first embodiment, the moving mechanism 91 is implemented differently from the first embodiment. Specifically, the path of a wire (the interlocking member 92) posterior to the interlocking member guide part 93 is different in the present embodiment. That is, the wire (the interlocking member 92) posterior to the interlocking member guide part 93 in the present embodiment extends toward the upper (Y1) side in the height direction Y on a more forward (Z2) side in the front-rear direction Z than the thigh side link 4 (the thigh side link main body part 40), passes through a hole 43C provided in a midway portion of a thigh side extended part 43 via an opening 43D to be guided toward the rear (Z1) side in the front-rear direction Z, and further extends to a length adjustment mechanism 33 provided in the waist side link 3 as shown in Fig. 28(A).

The length adjustment mechanism 33 is provided for adjusting the length of the wire (the interlocking member 92). As shown in Fig. 28(A), the length adjustment mechanism 33 includes, for example, a winding part 33A capable of winding the wire (the interlocking member 92), and a knob part 33B. When the knob part 33B is rotated clockwise, for example, the winding part 33A winds the wire (the interlocking member 92). When the knob part 33B is rotated counterclockwise, on the other hand, the winding part 33A feeds the wire (the interlocking member 92).

As shown in Figs. 28(A) and 28(B), a segment extending from the opening 43D of the hole 43C on the front (Z2) side in the front-rear direction Z to the winding part 33A is defined as a segment N. If the segment length of the segment N when the relative angle θ of the thigh side link 4 with respect to the waist side link 3 is θ2 is defined as L2 and the segment length of the segment N when the relative angle θ is the reference relative angle θ1 is defined as L3, L2 < L3 holds. Thus, when the relative angle θ of the thigh side link 4 with respect to the waist side link 3 is equal to the reference relative angle θ1, the wire (the interlocking member 92) is pulled by the winding part 33A, and the connected body 90 (not shown) is thereby moved toward the upper (Y1) side in the height direction Y (see Figs. 25 and 26). Consequently, the lower leg side link 6 swings with respect to the thigh side link 4 using the knee joint side swinging shaft 7 as an axis to assume the knee bending posture as shown in Fig. 27(C).

When the relative angle θ of the thigh side link 4 with respect to the waist side link 3 is equal to the relative angle θ2, on the other hand, the winding part 33A pulls no wire (interlocking member 92), and the connected body 90 (not shown) is thus unmoved. Or, even if the winding part 33A pulls the wire (the interlocking member 92), the connected body 90 (not shown) moves only by a distance smaller than or equal to L1 (see Figs. 24(A) and 24(B)). Consequently, the lower leg side protruded part 61 (the lower leg side link 6) is pressed by the biasing part 88 of the stopper mechanism 85 to the knee extension position, and the lower leg side link 6 assumes the knee extension posture with respect to the thigh side link 4 as shown in Figs. 27(A) and 27(B). Note that the first regulation side link 82 and the second regulation side link 84 assume the braking posture when the winding part 33A pulls no wire (interlocking member 92).

As described above, since a path length in the segment N between a pass point (e.g., the opening 43D) of the wire (the interlocking member 92) and a connection point with the trailing end of the wire (the interlocking member 92) varies between before and after relative swinging of the thigh side link 4 with respect to the waist side link 3, the wire (the interlocking member 92) is pulled and the connected body 90 is thereby moved toward the upper side. Other aspects are also included in the present invention as long as, in such an aspect: a pass point (e.g., the opening 43D) at which the wire (the interlocking member 92) passes through the thigh side link 4 from the front (Z2) side to the rear (Z1) side in the front-rear direction Z is provided at a position different from the hip joint side swinging shaft 5 (e.g., on the upper (Y1) side or the lower (Y2) side in the height direction Y than the hip joint side swinging shaft 5); the wire (the interlocking member 92) is fixed to any of the parts operating along with the waist side link 3 on a more rearward (Z1) side in the front-rear direction Z than the thigh side link 4; and a path length in the segment N becomes longer when the relative angle θ of the thigh side link 4 with respect to the waist side link 3 becomes smaller than the reference relative angle θ1.

Note that the walking assistance device 1 and the walking assistance mechanism 1A of the present invention are not limited to the above-described embodiments. It is apparent that various modifications can be made thereto without departing from the gist of the present invention. Note that those obtained by suitably combining the features of the first embodiment and the second embodiment of the present invention as desired can be also included in the scope of the present invention.

### Reference Signs List

- 1: walking assistance device
- 1A: walking assistance mechanism
- 2: wearing part
- 3: waist side link
- 4: thigh side link
- 5: hip joint side swinging shaft
- 6: lower leg side link
- 7: knee joint side swinging shaft
- 8: knee extension posture maintenance mechanism
- 9: knee bending posture allowance mechanism
- 10: walking posture ensuring part
- 20: first belt part
- 21: second belt part
- 22: lower back side belt portion
- 23: first belly side belt portion
- 24: second belly side belt portion
- 25: waist side belt part
- 26: crotch side belt part
- 27: connecting mechanism
- 27A: first connection part
- 27B: second connection part
- 30: base link
- 31: assist part
- 31A: grip portion
- 31B: assist part side depressed portion
- 31C: upper portion
- 33: length adjustment mechanism
- 33A: winding part
- 33B: knob part
- 40: thigh side link main body part
- 41: first thigh side protruded part
- 42: second thigh side protruded part
- 43, 43A, 43B: thigh side extended part
- 43C: hole
- 43D: opening
- 44, 44A, 44B: leading end portion
- 45: protruded part
- 46: wire facing surface
- 47: depressed portion
- 47A: depressed portion
- 60: lower leg side link main body part
- 61: lower leg side protruded part
- 61A: contact surface
- 64: guide groove
- 70: lever member
- 75: engagement member
- 80: lower leg side regulation shaft
- 81: thigh side regulation shaft
- 82: first regulation side link
- 83: intermediate regulation shaft
- 84: second regulation side link
- 85: stopper mechanism
- 87: guide part
- 88: biasing part
- 89: moving range limiting part
- 89A: contact part
- 90: connected body
- 90A: connection part
- 90AA: leading end surface
- 90B: guide side engagement part
- 90C: through hole
- 90CA: end face
- 90D: lower end portion
- 91: moving mechanism
- 92: interlocking member
- 93: interlocking member guide part
- 100: waist side connecting member
- 110: bridging link
- 120: bridging link guide part
- 121: lateral side guide portion
- 122: back side guide portion
- 123: extended part side swinging shaft
- 210: strip-shaped body
- 212: strip-shaped body holding part
- 213: seventh connection part
- 214: eighth connection part
- 260: hip side support portion
- 260A: first branch portion
- 260B: second branch portion
- 261: first crotch support portion
- 262: second crotch support portion
- 263A: third connection part
- 263B: fourth connection part
- 263C: fifth connection part
- 263D: sixth connection part
- 264, 265: hole
- 880: cylinder
- 881: piston
- 882: rod
- 883: first biasing side shaft
- 884: second biasing side shaft
- 900: user
- 910: waist portion
- 920: thigh portion
- 930: hip joint
- 940: lower leg portion
- 950: knee joint
- 960A,: 960B leg
- 970: crotch portion
- 971: hip
- 973: right leg
- 974: left leg

## Claims

1. A walking assistance mechanism for assisting walking of a user in a front-rear direction, comprising:
a waist side link disposed near a waist portion of the user;
a thigh side link disposed along a thigh portion of the user and connected to the waist side link to be able to relatively swing in the front-rear direction of the user by means of a hip joint side swinging shaft;
a lower leg side link disposed along a lower leg portion of the user and connected to the thigh side link to be able to relatively swing in the front-rear direction by means of a knee joint side swinging shaft;
a knee extension posture maintenance mechanism configured to regulate relative swinging between the thigh side link and the lower leg side link to maintain a knee extension posture in which a relative angle between the thigh side link and the lower leg side link is large; and
a knee bending posture allowance mechanism configured to release the regulation of the knee extension posture maintenance mechanism to allow for a knee bending posture in which the relative angle between the thigh side link and the lower leg side link becomes smaller when the thigh side link is positioned on a more rearward side than a position at which the thigh side link forms a predetermined reference relative angle with respect to the waist side link.

2. The walking assistance mechanism according to claim 1, wherein
the knee extension posture maintenance mechanism includes:
a lower leg side regulation shaft that is provided at a position away from the knee joint side swinging shaft in the lower leg side link and that is configured to move integrally with the lower leg side link;
a thigh side regulation shaft that is provided at a position away from the knee joint side swinging shaft in the thigh side link and that is configured to move integrally with the thigh side link;
a first regulation side link provided to be able to relatively swing with respect to the lower leg side link using the lower leg side regulation shaft as a swinging axis;
an intermediate regulation shaft provided at a position away from the lower leg side regulation shaft in the first regulation side link;
a second regulation side link that is provided to be able to relatively swing with respect to the thigh side link using the thigh side regulation shaft as a swinging axis and that is connected to the first regulation side link via the intermediate regulation shaft to be able to relatively swing; and
a stopper mechanism configured to delimit an upper limit of a relative opening angle between the first regulation side link and the second regulation side link to a braking posture in which the relative opening angle, representing a degree of opening between the first regulation side link and the second regulation side link using the intermediate regulation shaft as a reference, is 180 degrees, or larger than or equal to 180 degrees,
a four bar linkage is constituted by the knee joint side swinging shaft, the lower leg side regulation shaft, the thigh side regulation shaft, and the intermediate regulation shaft,
the thigh side link and the lower leg side link assume a knee extension posture when the first regulation side link and the second regulation side link assume the braking posture, and
the thigh side link and the lower leg side link assume a knee bending posture when the relative opening angle between the first regulation side link and the second regulation side link becomes smaller than 180 degrees.

3. The walking assistance mechanism according to claim 2, wherein the knee extension posture maintenance mechanism includes a biasing part configured to bias the first regulation side link and/or the second regulation side link in a direction in which the relative opening angle between the first regulation side link and the second regulation side link increases.

4. The walking assistance mechanism according to claim 2, wherein the knee extension posture maintenance mechanism includes a biasing part configured to bias the lower leg side link in a direction in which the thigh side link and the lower leg side link assume a knee extension posture.

5. The walking assistance mechanism according to any one of claims 2 to 4, wherein
the knee bending posture allowance mechanism includes:
a connected body provided in at least any one of the first regulation side link, the second regulation side link, and the intermediate regulation shaft; and
a moving mechanism configured to change the relative opening angle between the first regulation side link and the second regulation side link to be smaller than 180 degrees by moving the connected body.

6. The walking assistance mechanism according to claim 5, wherein the moving mechanism moves the connected body along with a change in a relative angle of the thigh side link with respect to the waist side link.

7. The walking assistance mechanism according to claim 6, wherein
the moving mechanism includes an interlocking member with one thereof being connected to the waist side link and the other thereof being connected to the connected body, and
when the thigh side link is positioned on a more rearward side than the position at which the thigh side link forms the predetermined reference relative angle with respect to the waist side link, the interlocking member moves the connected body to make the relative opening angle between the first regulation side link and the second regulation side link smaller than 180 degrees.

8. The walking assistance mechanism according to claim 7, wherein the interlocking member is a wire, and the connected body is moved as a result of the wire pulling the connected body.

9. The walking assistance mechanism according to claim 7 or 8, wherein
a contact part configured to change a path of the interlocking member by coming into contact with the interlocking member is disposed in a manner radially displaced from the hip joint side swinging shaft, and
the interlocking member is relatively moved with respect to the thigh side link as a result of the contact part moving closer to or farther away from the waist side link along with a relative swinging operation between the waist side link and the thigh side link, and the path of the interlocking member is thereby changed.

10. The walking assistance mechanism according to claim 7 or 8, wherein
the moving mechanism includes a winding mechanism configured to wind the interlocking member along with a relative swinging operation between the waist side link and the thigh side link, and
the interlocking member is in a state wound by the winding mechanism when the thigh side link is positioned on a more rearward side than the position at which the thigh side link forms the predetermined reference relative angle with respect to the waist side link.

11. The walking assistance mechanism according to claim 10, wherein
the winding mechanism includes:
a lever member configured to rotate about the hip joint side swinging shaft, to which one end of the interlocking member is fixed;
an engagement member configured to swing about the hip joint side swinging shaft together with the thigh side link and engage with the lever member; and
a rotation regulating part configured to regulate rotation of the lever member,
the lever member is rotated by being pressed by the swinging of the engagement member, and
when the rotation of the lever member is regulated by the rotation regulating part, the engagement member has a phase difference with the lever member, and the interlocking member is thereby wound by the lever member.

12. The walking assistance mechanism according to any one of claims 1 to 11, wherein
the lower leg side link includes a lower leg side regulation shaft guide part extending in a guide direction that approaches the thigh side link as moving toward an upper side from a lower side in a height direction of the user when the thigh side link and the lower leg side link assume a knee extension posture, and
the lower leg side regulation shaft engages with the lower leg side regulation shaft guide part in a manner movable along the guide direction.

13. The walking assistance mechanism according to any one of claims 1 to 12, comprising a wearing part to be worn in the waist portion of the user, and wherein
the wearing part includes:
a waist side belt part to be worn around the waist portion of the user; and
a crotch portion side belt part to be worn so as to support the user from a lower side in a crotch portion of the user.

14. A walking supporting device including the walking assistance mechanism according to any one of claims 1 to 13 one for each leg of the user, the walking supporting device comprising:
a connecting member configured to integrally connect the waist side links in the two walking assistance mechanisms;
a bridging link bridged between the two walking assistance mechanisms and connected to the two thigh side links; and
a bridging link guide part configured to guide vicinities of ends of the bridging link in the front-rear direction, wherein
a moving direction of one end of the bridging link becomes opposite to a moving direction of the other end of the bridging link along with operations of the respective walking assistance mechanisms in the front-rear direction.
